(19) Europäisches Patentamt
European Patent Office
Office européen des brevets

(11) Numéro de publication : **0 392 882 B1**

# (12) FASCICULE DE BREVET EUROPEEN

(45) Date de publication du fascicule du brevet : **28.10.92 Bulletin 92/44**

(51) Int. Cl.$^5$ : **A61K 7/42**, A61K 7/48, A61K 7/00

(21) Numéro de dépôt : **90400345.6**

(22) Date de dépôt : **08.02.90**

## (54) Utilisation et compositions cosmétiques contenant des diorganopolysiloxanes à fonction benzalmalonate pour la protection de la peau et des cheveux.

(30) Priorité : **15.02.89 FR 8901989**

(43) Date de publication de la demande : **17.10.90 Bulletin 90/42**

(45) Mention de la délivrance du brevet : **28.10.92 Bulletin 92/44**

(84) Etats contractants désignés : **AT BE CH DE DK ES FR GB GR IT LI NL SE**

(56) Documents cités :
**EP-A- 358 584**
**EP-A- 0 100 651**
**EP-A- 0 138 321**
**US-A- 4 405 469**

(73) Titulaire : **L'OREAL**
**14, Rue Royale**
**F-75008 Paris (FR)**

(72) Inventeur : **Forestier, Serge**
**16 Allée Ferdinand Buisson**
**F-77410 Claye-Souilly (FR)**
Inventeur : **Lang, Gérard**
**44, avenue Lacour**
**F-95210 Saint-Gratien (FR)**
Inventeur : **Richard, Hervé**
**48, rue de l'Ermitage**
**F-75020 Paris (FR)**

(74) Mandataire : **Casalonga, Axel et al**
**BUREAU D.A. CASALONGA - JOSSE**
**Morassistrasse 8**
**W-8000 München 5 (DE)**

**EP 0 392 882 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à l'utilisation en cosmétique, notamment en tant qu'agents filtrant le rayonnement UV, de diorganopolysiloxanes à fonction benzalmalonate ainsi qu'aux nouvelles compositions cosmétiques contenant ces composés, destinées à la protection de la peau et des cheveux.

On sait que les radiations lumineuses de longueurs d'onde comprises entre 280 nm et 400 nm permettent le brunissement de l'épiderme humain et que les rayons de longueurs d'onde comprises entre 280 et 320 nm, connues sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est donc intéressant de disposer de composés absorbant les rayons UV sur une large bande afin de pouvoir filtrer à la fois les rayons UV-A et UV-B.

On sait par ailleurs que les constituants entrant dans les préparations cosmétiques ne possèdent pas toujours une stabilité suffisante à la lumière et qu'ils se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique, et en particulier dans les huiles et graisses.

Il est également souhaitable d'assurer aux cheveux une bonne protection contre la dégradation photochimique afin d'éviter en particulier une décoloration ou un changement de nuance.

On sait, par ailleurs, greffer sur des chaînes de polymères carbonés synthétiques, de polymères naturels, d'hydrolysats de protéines ou de polyaminoamides, des restes de molécules ayant un effet filtre vis-à-vis du rayonnement UV; ces polymères greffés décrits par exemple dans les brevets français n° 2 197 023, 2 237 912, 2 531 960, 2 548 018, 2 549 069, 2 586 692 et 2 586 693 peuvent être utilisés pour préparer des compositions cosmétiques protectrices de l'épiderme humain ou anti-solaires. On a cependant constaté que ces polymères greffés sont généralement peu solubles dans les solvants cosmétiques usuels, notamment dans les supports gras, et qu'ils forment des films dont la structure est trop rigide.

Or, la demanderesse a découvert que certains diorganopolysiloxanes à fonction benzalmalonate présentaient, de manière étonnante, de bonnes propriétés cosmétiques associées à de bonnes propriétés filtrantes dans une large gamme de longueurs d'onde allant de 280 à 360 nm. Ils présentent notamment un excellent caractère liposoluble, ce qui les rend utilisables dans les supports gras utilisés en cosmétique. Outre leur bon pouvoir filtrant et leur bonne solubilité dans les corps gras et les solvants cosmétiques usuels, ces diorganopolysiloxanes à fonction benzalmalonate présentent une excellente stabilité chimique et photochimique et ont l'avantage d'apporter de la douceur à la peau et aux cheveux, par lesquels ils sont bien tolérés.

La présente invention a donc pour objet l'utilisation en cosmétique, en particulier en tant qu'agents filtrant le rayonnement UV de longueurs d'onde comprises entre 280 et 360 nm, de diorganopolysiloxanes à fonction benzalmalonate choisis parmi ceux de formule :

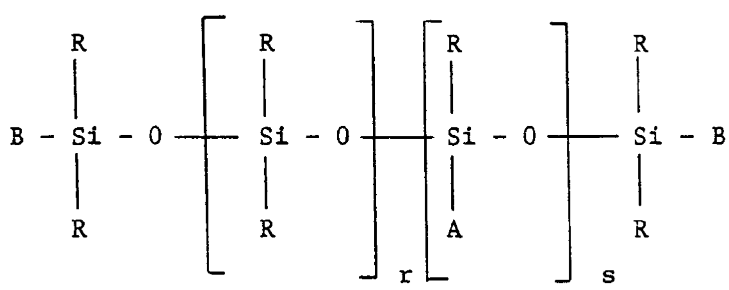

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A, et ceux de formule :

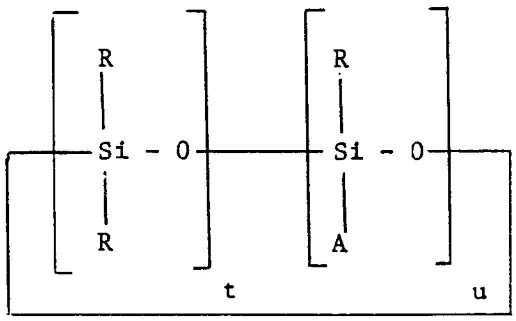

(2)

dans laquelle

R a la même signification qu'à la formule (1),

u est un nombre compris entre 1 et 20 inclus et

t est un nombre compris entre 0 et 20 inclus,

t + u est égal ou supérieur à 3,

formules dans lesquelles le symbole A est un radical de formule :

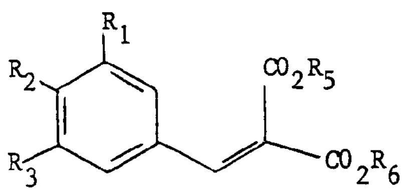

(3)

dans laquelle :

$R_1$ et $R_2$ sont choisis parmi un atome d'hydrogène, un radical hydroxyle, un radical triméthylsiloxy, un radical alkyle en $C_1$-$C_6$, un radical alcoxy en $C_1$-$C_6$, et un groupement divalent Y de formule :

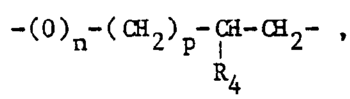

dans laquelle :

n est 0 ou 1, p est un nombre entier compris entre 1 et 10 inclus, de préférence entre 1 et 4, et $R_4$ est choisi parmi un atome d'hydrogène et un radical alkyle en $C_1$-$C_4$, l'un des deux radicaux $R_1$ et $R_2$ représentant nécessairement le groupement Y,

$R_3$ représente un atome d'hydrogène, un radical alkyle en $C_1$-$C_6$ ou un radical alcoxy en $C_1$-$C_6$.

$R_5$ et $R_5$, identiques ou différents, représentent un radical alkyle en $C_1$-$C_8$.

Dans les formules ci-dessus les radicaux alkyle et alcoxy peuvent être linéaires ou ramifiés.

Parmi les radicaux alcoxy en $C_1$-$C_6$ à chaîne droite ou ramifiée, on peut citer par exemple les radicaux méthoxy, éthoxy, n-propoxy, isopropoxy, n-butoxy, isobutoxy, tert.-butoxy, n-amyloxy, isoamyloxy, néopentyloxy et n-hexyloxy.

Parmi les radicaux alkyle en $C_1$-$C_6$ à chaîne droite ou ramifiée, on peut citer plus particulièrement les radicaux méthyle, éthyle, n-propyle, isopropyle, n-butyle, isobutyle, tert.-butyle, n-amyle, isoamyle, néopentyle et n-hexyle, et parmi les radicaux alkyle en $C_1$-$C_8$, les radicaux précédents ainsi que les radicaux n-heptyle, n-octyle et 2-éthylhexyle.

Les radicaux R alkyle préférés sont méthyle, éthyle, propyle, n-butyle, n-octyle et 2-éthylhexyle. De préférence au moins 80% en nombre des radicaux sont méthyle.

On préfère plus particulièrement les polymères statistiques ou à blocs de formules (1) et (2) présentant au moins l'une des caractéristiques suivantes :

- R est méthyle,
- B est méthyle,

- $R_1$ est H ou Y
- $R_2$ est Y, méthoxy ou butoxy
- $R_3$ est H ou méthoxy
- n = 0 ou 1
- p = 1
- $R_4$ est H ou méthyle
- $R_5$ et $R_6$ sont éthyle ou 2-éthylhexyle
- r est compris entre 5 et 20 inclus,
- s est compris entre 2 et 15 inclus,
- t + u est compris entre 3 et 10 inclus,

Pour préparer les polymères de formules (1) et (2), on peut par exemple partir du polymère correspondant dans lequel tous les radicaux A sont des atomes d'hydrogène.

Ce polymère est dénommé par la suite polymère à SiH; les groupes SiH peuvent être présents dans la chaîne et/ou aux extrémités de chaîne. Ces polymères à SiH sont des produits bien connus dans l'industrie des silicones et sont généralement disponibles dans le commerce.

Ils sont par exemple décrits dans les brevets américains US-A-3 220 972, US-A-3 436 366, US-A-3 697 473 et US-A-4 340 709.

Ce polymère à SiH peut être donc choisi parmi ceux de formule :

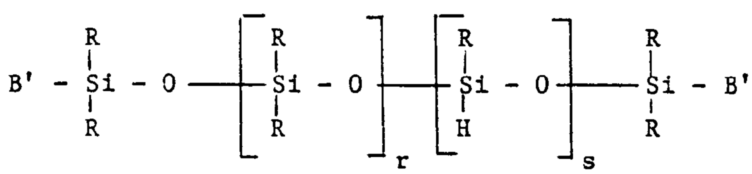

(4)

dans laquelle R, r et s ont la signification donnée ci-dessus pour la formule (1) et les radicaux B′, identiques ou différents, sont choisis parmi les radicaux R et un atome d'hydrogène, et de formule :

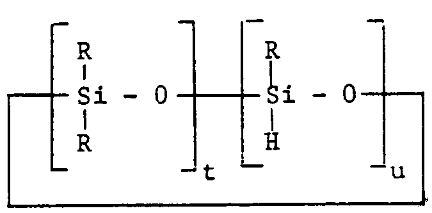

(5)

dans laquelle R, t et u ont la signification donnée ci-dessus pour la formule (2).

Sur ce polymère à SiH de formule (4) ou (5), on effectue une réaction d'hydrosilylation en présence d'une quantité catalytiquement efficace d'un catalyseur au platine sur un dérivé organique de benzalmalonate choisi parmi ceux de formule :

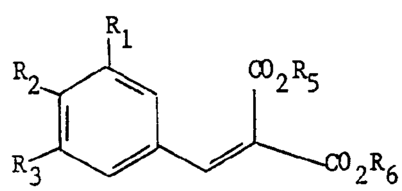

(6)

dans laquelle $R_1$, $R_2$, $R_3$, $R_5$ et $R_5$ ont la même signification qu'à la formule (3) ci-dessus, sauf que le radical Y est alors le radical homologue insaturé Y′ monovalent de formule :

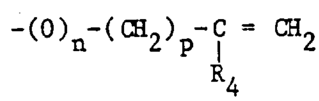

dans laquelle n, p et $R_4$ ont la même signification qu'à la formule (3) ci-dessus.

Certains des dérivés de formule (6) dans laquelle n = 1 sont déjà décrits dans la littérature de chimie, en particulier dans J. Chem. Soc. PERKIN TRANS (I) - 1985, pages 1627-1635 et dans Chem. Ber. Vol 99 - pages 1962-1965.

De façon générale les dérivés de formule (6) peuvent être préparés par réaction de KNOEVENAGEL, à savoir condensation d'un aldéhyde aromatique (II) avec un diester d'acide malonique de formule (III) dans le toluène en présence d'acétate de pipéridinium comme catalyseur. L'eau est éliminée par azéotropie. Le schéma réactionnel est le suivant :

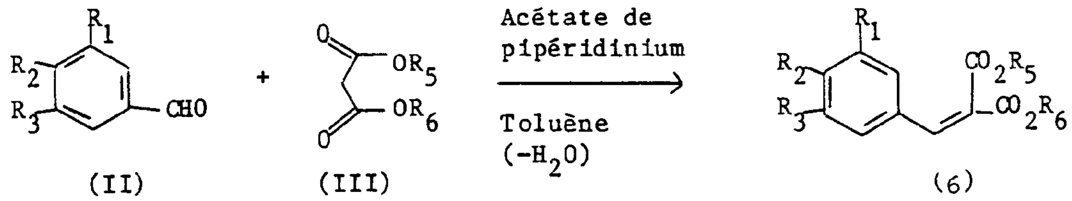


(II) (III) (6)

$R_1$, $R_2$, $R_3$, $R_5$ et $R_6$ ayant les significations indiquées ci-dessus pour la formule (6).

Les produits sont recristallisés, distillés ou séparés par chromatographie sur colonne. Les aldéhydes de formule (II), qui sont des composés connus, peuvent être obtenus selon l'une des méthodes suivantes :

<u>Première méthode</u>

L'aldéhyde de formule (II) dans laquelle $R_1$ représente un reste $-(CH_2)_p-C(R_4)=CH_2$ lorsque p = 1, $R_2$ représente un reste hydroxyle et $R_3$ a la signification mentionnée ci-dessus pour la formule (6), peut être obtenu par réarrangement de CLAISEN d'un aldéhyde de formule (IV) selon le schéma réactionnel suivant :

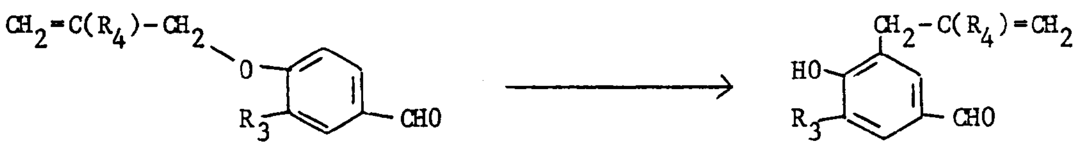

(IV) (IIA)

Ce réarrangement peut être effectué dans les conditions décrites par TARBELL (Organic Reactions, vol. 2, John Wiley, New York, 1944, page 1) par chauffage à au moins 170°C environ du composé de formule (IV), éventuellement en présence d'un solvant.

L'aldéhyde de formule (IV) peut être obtenu par réaction d'un halogénure d'alcényle de formule (V) sur un aldéhyde de formule (VI) :

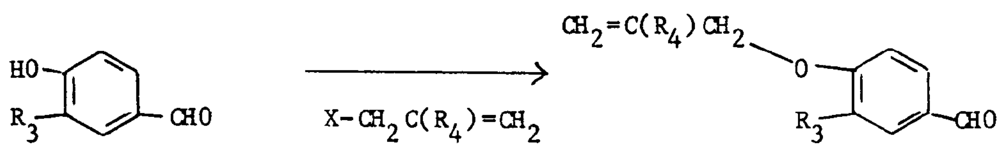


(VI) (V) (IV)

Cette réaction est effectuée en présence d'une base dans un solvant, par exemple en présence d'un carbonate de métal alcalin dans le diméthylformamide, à une température comprise entre la température ambiante et le point d'ébullition du solvant. L'aldéhyde de formule (VI) peut être préparé selon des méthodes connues. Dans le composé de formule (V), X représente un atome d'halogène, de préférence un atome de chlore ou de brome.

<u>Deuxième méthode</u>

L'aldéhyde de formule (IIB) répondant à la formule II dans laquelle $R_1$ représente un reste $-(CH_2)_p-C(R_4)=CH_2$ lorsque p = 1, $R_2$ représente un reste alcoxy en $C_1$-$C_6$ et $R_3$ a la signification mentionnée ci-dessus pour la formule (6), peut être obtenu selon l'une des deux voies ci-dessous :

Première voie

Par formylation d'un éther de phénol de formule (VII) selon le schéma réactionnel suivant :

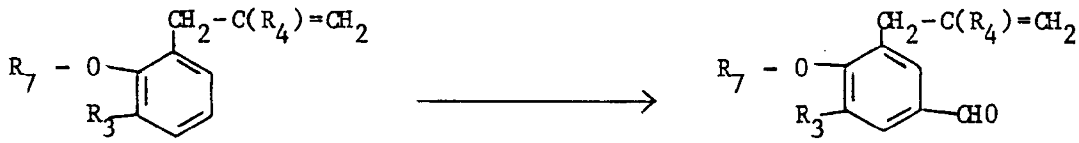

(VII) (IIB)

$R_7$ représente un radical alkyle en $C_1$-$C_6$, $R_3$ ayant la signification mentionnée ci-dessus.

Cette réaction est effectuée par exemple grâce à l'addition des complexes formés par l'action de l'oxychlorure de phosphore sur les formamides disubstitués selon VILSMEIER et HAACK (Ber., 60, p 119, 1927), sur les composés de formule (VII).

L'éther de phénol (VII) peut être préparé selon des méthodes connues.

Deuxième voie

Le composé de formule ($II_A$) obtenu par la première méthode peut être transformé en composé de formule (IIB) par réaction avec un halogénure ou un sulfate d'alkyle en $C_1$-$C_6$ en présence d'une base, par exemple en présence d'un carbonate de métal alcalin, dans un solvant tel que le diméthylformamide, ou bien en présence d'un hydrure de métal alcalin dans le 1,2-diméthoxyéthane, selon le schéma réactionnel suivant :

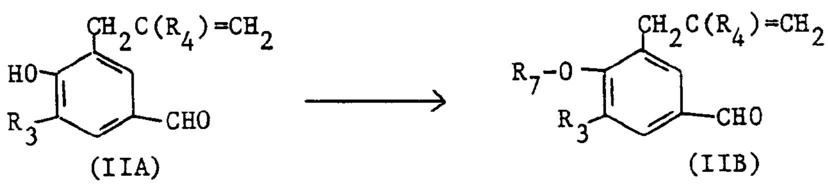

(IIA) (IIB)

Dans le composé de formule (IIB), $R_7$ représente un reste alkyle en $C_1$-$C_6$.

Troisième méthode

L'aldéhyde de formule (II) dans laquelle $R_1$ ou $R_2$ représente un reste $-(CH_2)_p-C(R_4)=CH_2$ et $R_3$ représente un atome d'hydrogène, un reste alkyle en $C_1$-$C_6$ ou un reste alcoxy en $C_1$-$C_6$ peut également être obtenu par réaction de l'orthoformiate d'éthyle sur un bromure de phénylmagnésium de formule (VIII), suivie d'une hydrolyse de l'acétal formé :

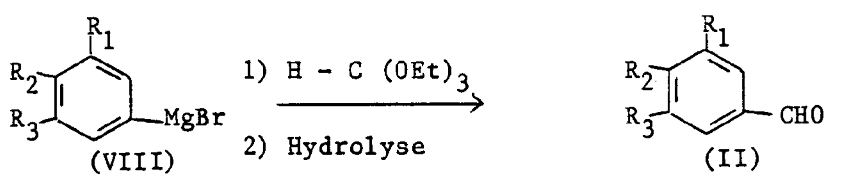


(VIII) (II)

Cette réaction peut être effectuée dans les conditions décrites par QUELET (C.R. Acad. Sci. vol. 182, p 1285 et Bull. Soc. Chim. Fr. vol.45 p 267), par exemple dans un solvant inerte tel que l'éther éthylique, le dioxane ou le 1,2-diméthoxyéthane, à une température comprise entre la température ambiante et le point d'ébullition du solvant. Dans les composés de formule (II) et (VIII), l'un des substituants $R_1$ ou $R_2$ représente un radical $-(CH_2)_p-C(R_4)=CH_2$, $R_4$ et p ayant les significations mentionnées ci-dessus, et l'autre représente un atome d'hydrogène, un radical alkyle en $C_1$-$C_6$ ou un radical alcoxy en $C_1$-$C_6$ et $R_3$ représente un atome d'hydrogène, un radical alkyle en $C_1$-$C_6$ ou un radical alcoxy en $C_1$-$C_6$.

Les catalyseurs au platine utilisés pour réaliser la réaction d'hydrosilylation des polymères de formule (4) ou (5) sur le dérivé organique de formule (6) sont amplement décrits dans la littérature. On peut en particulier citer les complexes du platine et d'un produit organique décrit dans les brevets américains US-A-3 159 601,

US-A-3 159 602, US-A-3 220 972 et les brevets européens EP-A-57 459, EP-A-188 978 et EP-A-190 530 et les complexes du platine et d'organopolysiloxane vinylé décrits dans les brevets américains US-A-3 419 593, US-A 3 377 432 et US-A-3 814 730.

Pour faire réagir le polymère à SiH de formule (4) ou (5) sur le dérivé de formule (6), on utilise généralement une quantité de catalyseur au platine, calculée en poids de platine métal, comprise entre 5 et 600 ppm, de préférence entre 10 et 200 ppm, basée sur le poids de polymère à SiH de formule (4) ou (5).

La réaction d'hydrosilylation peut avoir lieu en masse ou au sein d'un solvant organique volatil tel que le toluène, l'heptane, le xylène, le tétrahydrofuranne et le tétrachloréthylène.

Il est généralement souhaitable de chauffer le mélange réactionnel à une température de 60 à 120°C pendant le temps nécessaire pour que la réaction soit complète. On peut ajouter goutte à goutte le polymère à SiH sur le dérivé de formule (6) en solution dans un solvant organique contenant le catalyseur. On peut aussi ajouter simultanément le polymère à SiH et le dérivé de formule (6) à une suspension de catalyseur dans un solvant organique.

Il est recommandé de vérifier que la réaction est complète en dosant les SiH résiduels par la potasse alcoolique, puis on élimine le solvant, par exemple par distillation sous pression réduite.

L'huile brute obtenue peut être purifiée, par exemple par passage sur une colonne absorbante de silice.

Un autre objet de l'invention est constitué par les compositions cosmétiques destinées à protéger la peau et les cheveux du rayonnement UV, contenant une quantité efficace d'un diorganopolysiloxane à fonction benzalmalonate de formule (1) ou (2), dans un milieu cosmétiquement acceptable.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (1) ou (2) contenu dans un support cosmétiquement acceptable comprenant au moins une phase grasse.

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un diorganopolysiloxane à fonction benzalmalonate de formule (1) ou (2) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses, alcooliques ou oléoalcooliques, d'émulsions telles qu'une crème ou un lait, de gels oléoalcooliques, alcooliques ou hydroalcooliques, de bâtonnets solides, ou être conditionnée en aérosol.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (1) ou (2) est présent dans des proportions en poids comprises entre 0,25 et 3% par rapport au poids total de la composition cosmétique protectrice de l'épiderme humain.

Comme solvant de solubilisation, on peut utiliser une huile, une cire et de façon générale tout corps gras, un monoalcool ou un polyol inférieur, un benzoate d'alcools en $C_{12}$-$C_{15}$ ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du composé de formule (1) ou (2), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles ou synthétiques, de lanoline, et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas d'une composition conditionnée en aérosol, on utilise les propulseurs classiques tels que les alcanes, les fluoroalcanes et les chlorofluoroalcanes.

La présente invention vise également les compositions cosmétiques anti-solaires contenant au moins un composé de formule (1) ou (2) et pouvant contenir d'autres filtres UV-B et/ou UV-A.

Dans ce cas, la quantité totale de filtres présents dans la composition anti-solaire, c'est-à-dire le composé de formule (1) ou (2) et éventuellement les autres filtres, est comprise entre 0,5 et 15% en poids par rapport au poids total de la composition anti-solaire.

Ces compositions anti-solaires se présentent sous les formes indiquées ci-dessus pour les compositions protectrices de l'épiderme humain.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de spray de coiffage, de laque pour cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 5% en poids de composé de formule (1) ou (2).

La présente invention vise également les compositions cosmétiques contenant au moins un composé de formule (1) ou (2) à titre d'agent de protection contre les rayons ultraviolets constituées par des compositions capillaires tels que les laques pour cheveux, les lotions de mise en plis éventuellement traitantes ou démêlantes, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,25 à 3% en poids de composé de formule (1) ou (2).

L'invention vise également un procédé de protection des compositions cosmétiques contre les rayons ultraviolets, consistant à incorporer à ces compositions une quantité efficace d'au moins un composé de formule (1) ou (2).

Les exemples ci-après illustrent l'invention sans en limiter la portée.

EXEMPLE 1

Préparation du polymère statistique de formule :

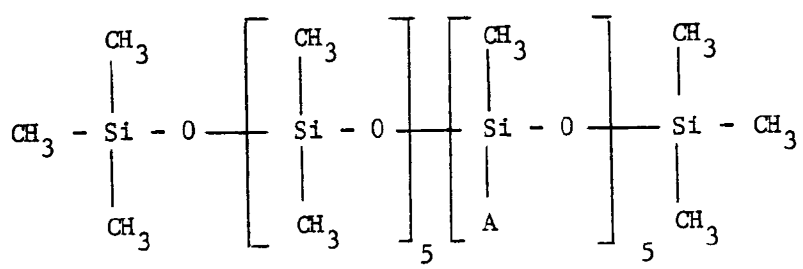

(1)

où A représente :

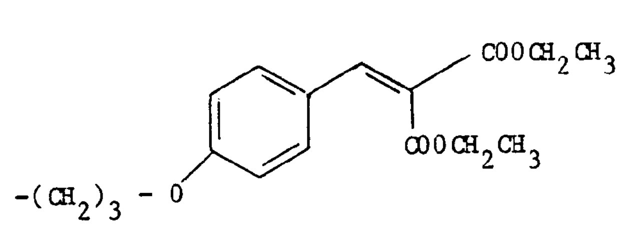

A une suspension de platine sur charbon à 5% (166 mg) dans du toluène sec (5 ml) à 90-100°C sous azote et sous agitation, on ajoute goutte à goutte en 1 heure 30 minutes, une solution dans du toluène (55 ml) de 30 g de 4-allyloxy benzal malonate de diéthyle et 16 g du polymère statistique de formule ci-dessus où A est un atome d'hydrogène, tout en maintenant la température entre 100 et 105°C.

On laisse sous agitation et au reflux jusqu'à disparition des groupements SiH (absence de bande à 2180cm$^{-1}$ en infrarouge), soit 10 heures. On filtre sur papier, on élimine le solvant et on lave deux fois à l'éthanol à 80%. L'huile obtenue est reprise dans le chloroforme, séchée sur sulfate de sodium et filtrée sur célite pour éliminer les restes de platine colloïdal. On obtient après évaporation du solvant une huile jaune pâle (poids : 36 g, rendement : 78%).

Spectre UV (éthanol) : λmax : 311 nm.

L'analyse par résonance magnétique nucléaire ($^{1}$H et $^{29}$Si RMN) indique que le produit a bien la structure souhaitée.

EXEMPLE 2

Préparation du polymère statistique de formule :

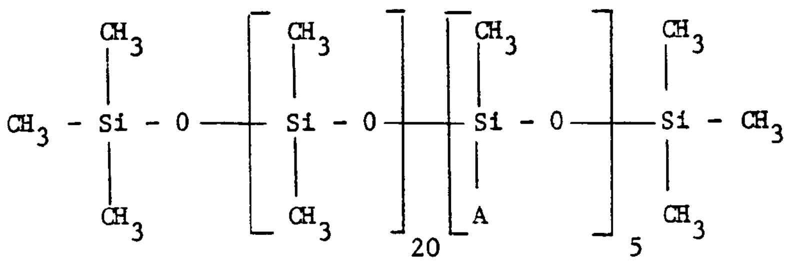

(1)

où A représente :

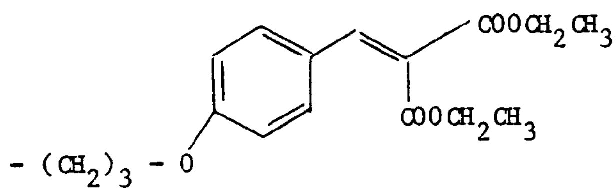

On répète le mode opératoire de l'exemple 1 en partant du polymère statistique de formule ci-dessus où A est un atome d'hydrogène.

On obtient ainsi une huile légèrement jaune.

Spectre UV ($CHCl_3$) : λmax = 313 nm

EXEMPLE 3

Préparation du polymère statistique de formule :

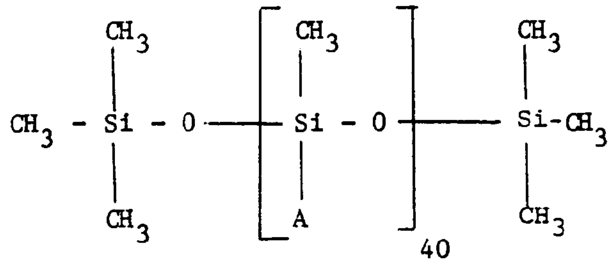

où A représente :

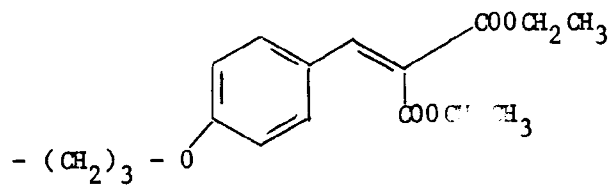

On répète le mode opératoire de l'exemple 1 en partant du polymère statistique de formule ci-dessus où A est un atome d'hydrogène.

On obtient une huile jaune visqueuse.

Spectre UV ($CHCl_3$) : λmax = 313 nm.

EXEMPLE 4

Préparation du polymère statistique de formule :

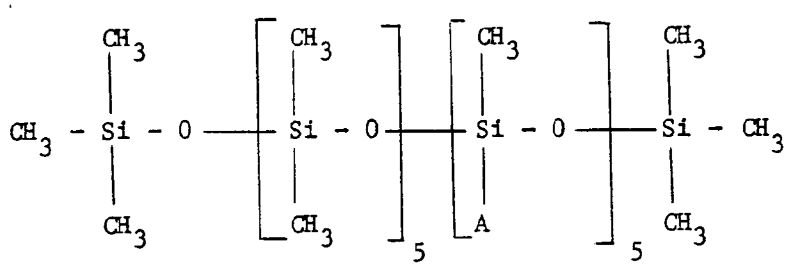 (1)

où A représente

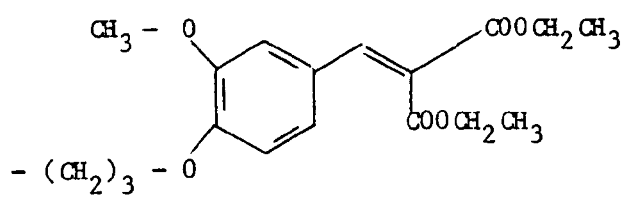

On répète le mode opératoire de l'exemple 1, sauf que l'on part de 20 g de 4-allyloxy-3-méthoxy benzalmalonate de diéthyle et de 9,7 g du polymère statistique de formule ci-dessus où A est un atome d'hydrogène.

On obtient 22 g d'une huile épaisse jaune pâle (rendement : 74%).

Spectre UV ($CHCl_3$) : $\lambda\ max_1 = 330$ nm
$\lambda\ max_2 = 300$ nm (épaulement).

L'analyse par résonance magnétique nucléaire ($^1H$ et $^{29}Si$) indique que le produit obtenu a bien la structure souhaitée.

EXEMPLE 5

Préparation du polymère statistique de formule :

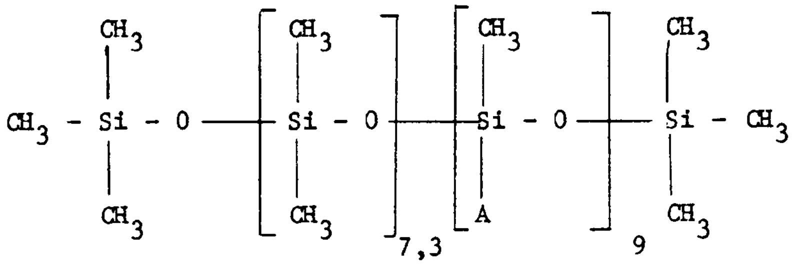

où A représente :

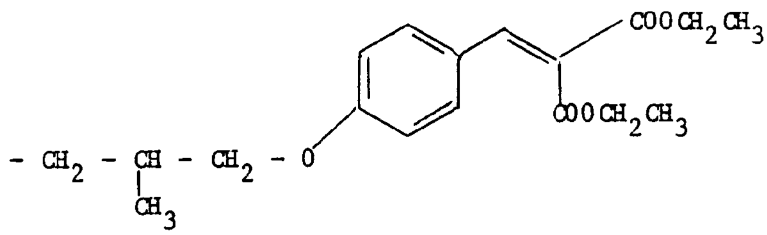

Dans un ballon tricol maintenu à 100°C par un bain d'huile muni d'une agitation magnétique et d'un réfrigérant ascendant, on charge 22,7 g (0,071 mole) de 4-méthallyloxy benzalmalonate de diéthyle, 33 g de toluène et 14,4,µl d'une solution dans l'hexane (à 8,45% en poids de platine métal) d'un complexe de platine préparé à partir d'acide chloroplatinique et de 1,3-divinyl 1,1,3,3-tétraméthyl disiloxane comme décrit dans le brevet US-A-3 814 730.

On ajoute en une heure 10 g du polymère statistique de formule ci-dessus où A est un atome d'hydrogène titrant 713 meq/100 g en fonction SiH (meq = milliéquivalent).

Au bout de 24 heures de réaction, on vérifie au moyen de potasse butanolique par dosage des SiH, que le taux de transformation des fonctions SiH est de 73%.

On obtient alors une huile trouble légèrement jaune, d'odeur agréable et de très forte viscosité, après avoir éliminé le toluène par distillation à 100°C sous pression réduite de 0,66 KPa.

Sur un échantillon de l'huile obtenue on effectue une analyse de Résonance Magnétique Nucléaire du proton (RMN$^1$H) à 360 MHz dans $CDCl_3$ qui montre l'existence du monomère non réagi, d'hydrogénosilane, et de la structure résultant d'hydrosilylation de l'insaturation méthallyloxy du monomère, soit :

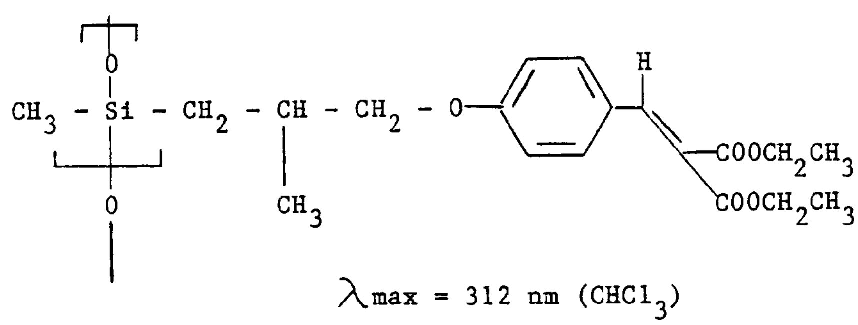

$\lambda$max = 312 nm ($CHCl_3$)

EXEMPLE 6

Exemple 6a : 3-allyl-4-méthoxy-benzalmalonate de diéthyle :

Préparation d'un composé de formule générale (6) dans laquelle $R_1$ représente le radical $-CH_2-CH = CH_2$, $R_2$ représente le radical $-OCH_3$, $R_3$ représente un atome d'hydrogène et $R_5$ et $R_5$ représentent le radical $-C_2H_5$ :

Premier stade : Preparation du 3-allyl-4-méthoxy-benzaldéhyde :

Première méthode

On chauffe pendant 4 heures sous azote et sous agitation à 220°C, 50 g (0,308 mole) de 4-allyloxybenzaldéhyde. Le mélange réactionnel refroidi est repris dans du dichlorométhane et extrait à la soude 5N. La phase aqueuse est acidifiée à l'acide chlorydrique 6N et extraite au dichlorométhane. La phase organique est séchée et le solvant évaporé pour donner une huile brun noir. Après distillation sous vide, on recueille la fraction Eb = 138-140°C sous 106 Pa (15 g, rendement : 30%) de 3-allyl-4-hydroxy-benzaldéhyde (poudre blanche, Pf = 66°C).

On introduit sucessivement le dérivé précédent (14,5 g, 0,089 mole), 30 ml de N,N-diméthylformamide, 13,6 g (0,098 mole) de carbonate de potassium anhydre et 11 ml (0,178 mole) d'iodure de méthyle. On porte le tout à 60-70°C pendant 3 heures. On verse le mélange réactionnel dans de l'eau glacée et on extrait le tout à l'éther diisopropylique. On sèche la phase organique sur sulfate de sodium, on filtre et évapore le solvant pour obtenir le 3-allyl-4-méthoxybenzaldéhyde (huile jaune pâle, 13,6 g, rendement = 87%).

Deuxième méthode

Dans un réacteur de 5 litres, on introduit successivement du 2-allylphénol (100 g, 0,75 mole), 2 litres de N,N-diméthylformamide sec et du carbonate de potassium anhydre (206 g, 1,49 mole). A température ambiante, on introduit goutte à goutte de l'iodure de méthyle (92 ml, 1,49 mole). On laisse pendant 4 heures à 38°C. On verse le mélange réactionnel dans de l'eau glacée et on extrait au dichlorométhane. La phase organique est lavée à l'eau et séchée. Après évaporation du solvant et distillation sous vide, on récupère une fraction distillant à 110°C sous 5000 Pa de 2-allylanisole (liquide incolore, 46 g, rendement = 42%).

Dans un réacteur de 500 ml, on place du N,N-diméthylformamide (75 ml, 0,98 mole) et on ajoute, tout en refroidissant vers 5°C, de l'oxychlorure de phosphore (26 ml, 0,28 mole). On maintient le mélange une heure à 10°C et on introduit goutte à goutte le dérivé précédent (41,5 g, 0,28 mole). On monte progressivement la température à 100°C en une heure et on maintient le mélange réactionnel à cette température pendant 10 heures. Le mélange refroidi est versé dans de l'eau glacée et extrait à l'éther diisopropylique. Les phases organiques sont lavées à l'eau, séchées sur sulfate de sodium, filtrées et le solvant évaporé pour donner un produit brut (31g), lequel est purifié par chromatographie sur silice 60 (éluant : toluène/hexane 50/50) pour donner une fraction (4,5 g) de 3-allyl-4-méthoxy-benzaldéhyde identique à celui obtenu par la première méthode.

Deuxième stade : préparation du 3-allyl-4-méthoxy-benzalmalonate de diéthyle

On chauffe au reflux, sous azote avec un Dean Stark, un mélange du dérivé précédent (10 g, 0,057 mole), de malonate de diéthyle (9,09 g, 0,057 mole), de toluène (15 ml), d'acide acétique (0,36 ml) et de pipéridine (0,68 ml). Après 5 heures de chauffage, on a recueilli 1 ml d'eau. Après refroidissement, on lave la phase toluénique à l'eau, on la sèche et on distille le solvant. On obtient une huile orangée qui cristallise. On la recristallise dans l'éther diisopropylique avec traitement au noir animal. On obtient des cristaux blancs de 3-allyl-4-méthoxy benzalmalonate de diéthyle (12,7 g, rendement = 70%) possédant les caractéristiques suivantes :

- point de fusion : 69°C,
- spectre de RMN $^1H$ ($CDCl_3$) : spectre conforme à la structure attendue,

- spectre UV ($CHCl_3$) : $\lambda$ max : 318 nm
  $\varepsilon$ : 24450

- analyse élémentaire :

calculé : C 67,91; H 6,97; O 25,13
trouvé: : C 68,04; H 6,89; O 25,23

Exemple 6b

Préparation du polymère statistique de formule :

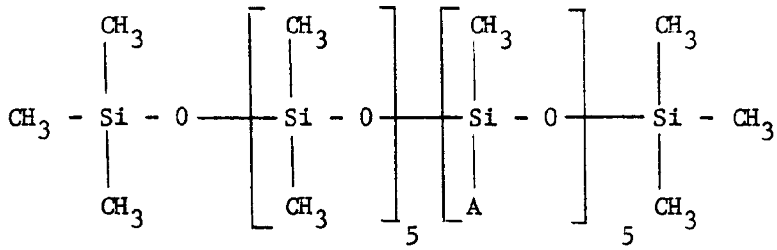

où A représente :

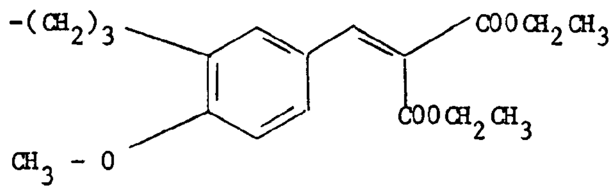

A une suspension de platine sur charbon à 5% (55 mg) dans du toluène sec (5 ml) à 90-100°C, sous azote et sous agitation, on ajoute goutte à goutte en 1 heure 30 minutes, une solution toluénique (20 ml) de 3-allyl-4-méthoxy benzalmalonate de diéthyle (10 g, 31,4 méq) et de polymère statistique de formule ci-dessus où A est un atome d'hydrogène (4,60 g, 31,4 méq en SiH) tout en maintenant la température entre 100 et 105°C. On laisse sous agitation et au reflux jusqu'à disparition des groupements SiH (absence de bande à 2180 $cm^{-1}$ en infrarouge), soit 8 heures. On filtre sur papier, on élimine le solvant et on lave deux fois à l'éthanol à 80%. L'huile jaune pâle obtenue est reprise dans le dichlorométhane, séchée sur sulfate de sodium et passée sur lit de silice 60. On obtient après évaporation du solvant une huile visqueuse jaune pâle (5,3 g, rendement = 36%).

| | |
|---|---|
| Spectre $^1H$ RMN ($CDCl_3$) | : spectre conforme à la formule, |
| Spectre $^{29}Si$ RMN ($CDCl_3$) | : spectre conforme à la formule, |
| Spectre UV ($CHCl_3$) | : $\lambda$ max : 318 nm. |

EXEMPLE 7

Exemple 7a : 3-allyl-4,5-diméthoxy-benzalmalonate de diéthyle :

Préparation d'un composé de formule générale (6) dans laquelle $R_1$ représente le radical $CH_2$-CH = $CH_2$, $R_2$ et $R_3$ représentent le radical $-OCH_3$ et $R_5$ et $R_6$ représentent le radical $-C_2H_5$.

Premier stade

On porte à 180°C pendant 6 heures 30 minutes sous agitation le 4-allyloxy-3-méthoxy-benzaldéhyde (62,5 g, 0,325 mole). On refroidit. Le solide marron est repris dans le dichlorométhane et extrait à la soude à 5%. La phase aqueuse est acidifiée à l'acide chlorhydrique 3N. Le solide obtenu est filtré et recristallisé dans un mélange éthanol/eau (40/60). On obtient le 3-allyl-4-hydroxy-5-méthoxy-benzaldéhyde (poudre beige clair, 62,5 g, rendement = 71%, point de fusion = 83-84°C).

Deuxième stade

On introduit successivement dans un réacteur le dérivé précédent (34 g, 0,18 mole), le diméthylformamide (500 ml), le carbonate de potassium (49 g, 0,35 mole) et l'iodure de méthyle (50 g, 0,35 mole). On maintient à une température de 40°C pendant 3 heures. On plonge le mélange réactionnel dans de l'eau glacée et on extrait l'huile formée par du dichlorométhane. Après lavage, séchage et évaporation du solvant, on obtient une huile brun clair, laquelle est passée sur lit de silice 60 pour donner une huile jaune pâle de 3-allyl-4,5-diméthoxy-benzaldéhyde (34,3 g, rendement = 92%).

Troisième stade :

On chauffe pendant 7 heures au reflux avec un Dean Stark un mélange du dérivé précédent (15 g, 0,073 mole), de malonate de diéthyle (11,7 g, 0,073 mole), de toluène (18 ml), d'acide acétique (0,46 ml) et de pipéridine (0,87 ml). Après refroidissement, on lave la phase toluénique à l'eau, on la sèche et on distille le solvant. L'huile orange pâle obtenue (24,5 g, rendement : 96%) est cristallisée dans un mélange éther diisopropylique/hexane (50/50) pour donner des cristaux blancs de 3-allyl-4,5-diméthoxy-benzalmalonate de diéthyle (14,2 g, rendement = 56%) possédant les caractéristiques suivantes :

- point de fusion : 43-44°C,
- spectre de RMN $^{1}H$ ($CDCl_3$) : spectre conforme à la formule attendue,
- spectre UV ($CHCl_3$) : $\lambda max_1$ = 303 nm  $\varepsilon$ = 15 700
  $\lambda max_2$ = 325 nm  $\varepsilon$ = 12 830
  (épaulement)
- analyse élémentaire :

calculé : C 65,50; H 6,94; O 27,55
trouvé : C 65,33; H 6,91; O 27,78

Exemple 7b

Préparation du polymère statistique de formule :

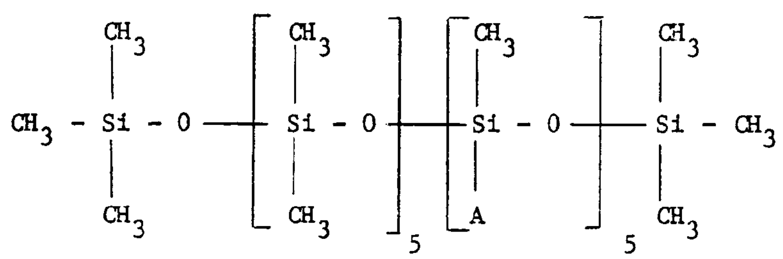

où A représente :

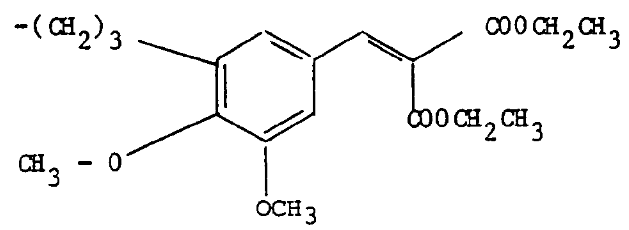

A une suspension de platine sur charbon à 5% (106 mg) dans du toluène sec (5 ml) à 90-100°C, sous azote et sous agitation, on ajoute goutte à goutte en 1 heure 30 minutes, une solution toluénique (30 ml) de 3-allyl-4,5-diméthoxy benzalmalonate de diéthyle (10 g, 28,7 méq) et de polymère statistique de formule ci-dessus où A est un atome d'hydrogène (4,55 g, 28 méq en SiH) tout en maintenant la température entre 100 et 105°C. On laisse sous agitation et au reflux jusqu'à disparition des groupements SiH (absence de bande à 2180 $cm^{-1}$ en infrarouge), soit 12 heures. On filtre sur papier, on élimine le solvant et on lave deux fois à l'éthanol à 80%. L'huile jaune pâle obtenue est reprise dans le dichlorométhane, séchée sur sulfate de sodium et passée sur lit de silice 60. On obtient après évaporation du solvant une huile visqueuse jaune pâle (10,6 g, rendement : 73%).

Spectre $^{1}H$ RMN ($CDCl_3$) : spectre conforme à la formule,
Spectre $^{29}Si$ RMN ($CDCl_3$) : spectre conforme à la formule,

Spectre UV ($CHCl_3$) : λ max : 304nm.

EXEMPLE 8

Exemple 8a : 3-allyl-4,5-diméthoxy-benzalmalonate de di-(2-éthylhexyle):

Préparation d'un composé de formule générale (6) dans laquelle $R_1$ représente le radical $-CH_2-CH=CH_2$, $R_2$ et $R_3$ représentent le radical $-OCH_3$ et $R_5$ et $R_6$ représentent le radical $-CH_2CH(C_2H_5)C_4H_9$.

On chauffe au reflux pendant 5 heures avec un Dean Stark un mélange de 3-allyl-4,5-diméthoxy-benzaldéhyde (10,3 g, 0,05 mole), de malonate de di-(2-éthylhexyle) (16,4 g, 0,05 mole), de toluène (20 ml), d'acide acétique (0,41 ml) et de pipéridine (0,77 ml). Après refroidissement, lavage à l'eau de la phase toluénique, séchage et évaporation du solvant, on obtient une huile orangée laquelle est purifiée par chromatographie sur colonne de silice 60 (éluant : heptane/acétate d'éthyle 90/10) pour donner le 3-allyl-4,5-diméthoxy-benzalmalonate de di-(2-éthylhexyle) (huile incolore, 15 g, rendement = 64%) possédant les caractéristiques suivantes :

- spectre de RMN $^1H$ ($CDCl_3$) : spectre conforme à la formule attendue,

- spectre UV ($CHCl_3$) : $\lambda\ max_1 = 303$ nm $\varepsilon = 15\ 500$
  $\lambda\ max_2 = 320$ nm $\varepsilon = 13\ 430$ (épaulement)

- analyse élémentaire :

calculé : C 72,06; H 9,36; O 18,58
trouvé : C 72,09; H 9,44; O 18,69

Exemple 8b

Préparation du polymère statistique de formule :

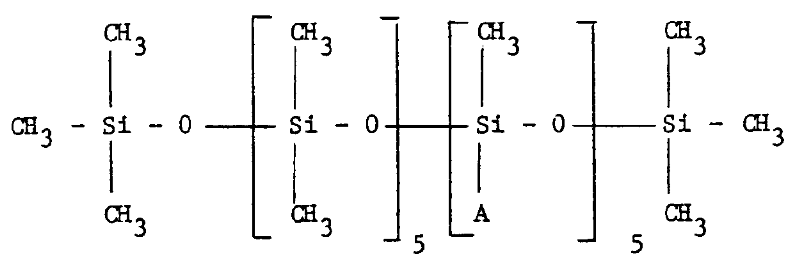

où A représente :

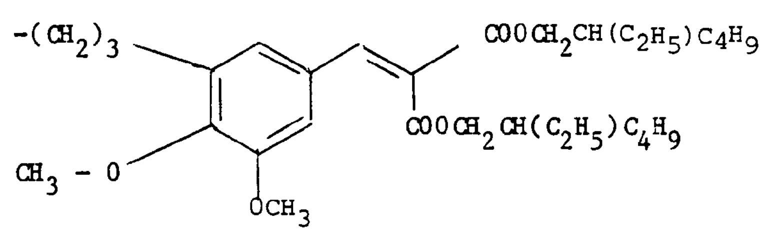

A une suspension de platine sur charbon à 5% (80 mg) dans du toluène sec (5 ml) à 90-100°C, sous azote et sous agitation, on ajoute goutte à goutte en 1 heure 30 minutes, une solution toluénique (30 ml) de 3-allyl-4,5-diméthoxy-benzalmalonate de di-(2-éthyl-hexyle) (10 g, 19,3 méq) et de polymère statistique de formule ci-dessus où A est un atome d'hydrogène (2,86 g, 17,6 méq en SiH) tout en maintenant la température entre 100 et 105°C. On laisse sous agitation et au reflux jusqu'à disparition des groupements SiH (absence de bande à 2180 $cm^{-1}$ en infrarouge), soit 12 heures. On filtre sur papier, on élimine le solvant et on lave deux fois à l'éthanol à 80%. L'huile jaune pâle obtenue est reprise dans le dichlorométhane, séchée sur sulfate de sodium

et passée sur lit de silice 60. On obtient après évaporation du solvant une huile visqueuse jaune pâle (5,1 g, rendement = 43%).

Spectre $^{1}H$ RMN ($CDCl_3$) : spectre conforme à la formule,
Spectre $^{29}Si$ RMN ($CDCl_3$) : spectre conforme à la formule,
Spectre UV ($CHCl_3$) : $\lambda$ max : 305 nm

## EXEMPLE 9

### Exemple 9a : 3-allyl-4-butoxy-5-méthoxy-benzalmalonate de diéthyle

Préparation d'un composé de formule générale (6) dans laquelle $R_1$ représente le radical $-CH_2CH = CH_2$, $R_2$ représente le radical $-OC_4H_9$, $R_3$ représente le radical $-OCH_3$ et $R_5$ et $R_6$ représentent le radical $-C_2H_5$.

Premier stade :

On maintient pendant 3 heures à 40-45°C un mélange de 3-allyl-4-hydroxy-5-méthoxy-benzaldéhyde (10,25 g, 0,053 mole), de diméthylformamide (150 ml), de carbonate de potassium (8,29 g, 0,06 mole) et de 1-bromobutane (8,22 g, 0,06 mole). On plonge le mélange réactionnel dans de l'eau glacée et on extrait l'huile formée par du dichlorométhane. Après lavage à l'eau, séchage et évaporation du solvant, on obtient une huile brune laquelle est passée sur lit de silice 60 pour donner une huile jaune pâle de 3-allyl-4-butoxy-5-méthoxy-benzaldéhyde (13 g, rendement = 91%).

Deuxième stade :

On chauffe au reflux pendant 7 heures avec un Dean Stark un mélange du dérivé précédent (10,2 g, 0,041 mole), de malonate de diéthyle (7 g, 0,041 mole), de toluène (12 ml), d'acide acétique (0,26 ml) et de pipéridine (0,49 ml). De la même manière qu'à l'exemple 8a, on obtient le 3-allyl-4-butoxy-5-méthoxy-benzalmalonate de diéthyle (huile incolore, 10 g, rendement = 67%) possédant les caractéristiques suivantes :

- spectre de RMN $^{1}H$ ($CDCl_3$) : spectre conforme à la formule attendue,
- spectre UV ($CHCl_3$) : $\lambda\ max_1 = 305$ nm $\varepsilon = 15\ 500$
  $\lambda\ max_2 = 325$ nm $\varepsilon = 13\ 530$
  (épaulement)
- analyse élémentaire :

calculé : C 67,67; H 7,74; O 24,58
trouvé : C 67,87; H 7,83; O 24,44

### Exemple 9b

Préparation du polymère statistique de formule :

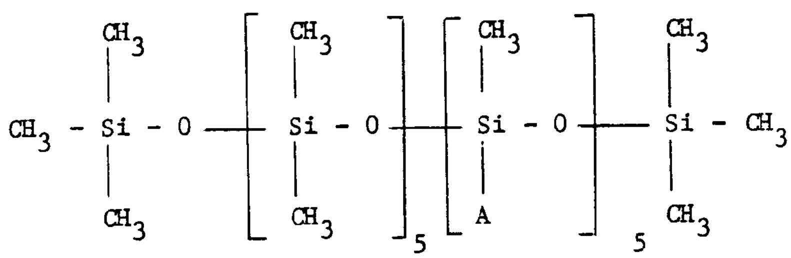

où A représente :

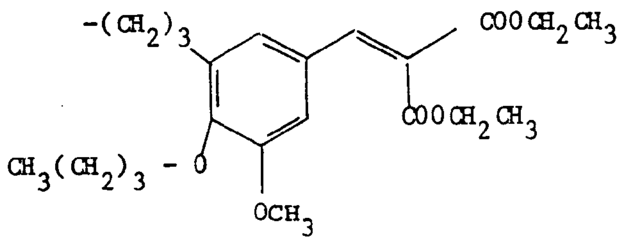

A une suspension de platine sur charbon à 5% (60 mg) dans du toluène sec (5 ml) à 90-100°C, sous azote et sous agitation, on ajoute goutte à goutte en 1 heure 30 minutes, une solution toluénique (20 ml) de 3-allyl-4-butoxy-5-méthoxy benzalmalonate de diéthyle (8,2 g, 21 méq) et de polymère statistique de formule ci-dessus où A est un atome d'hydrogène (3,24 g, 19,9 méq en SiH) tout en maintenant la température entre 100 et 105°C. On laisse sous agitation et au reflux jusqu'à disparition des groupements SiH (absence de bande à 2180 $cm^{-1}$en infrarouge), soit 10 heures. On filtre sur papier, on élimine le solvant et on lave deux fois à l'éthanol à 80 %. L'huile jaune pâle obtenue est reprise dans le dichlorométhane, séchée sur sulfate de sodium et passée sur lit de silice 60. On obtient après évaporation du solvant une huile visqueuse incolore (4,7 g, rendement = 43%).

- Spectre $^1H$ RMN ($CDCl_3$) : spectre conforme à la formule,
- Spectre $^{29}Si$ RMN ($CDI_3$) : spectre conforme à la formule,
- Spectre UV ($CHCl_3$) : $\lambda$ $max_1$: 306 nm.
  $\lambda$ $max_2$: 325 nm.

EXEMPLES D'APPLICATION

Exemple A : Emulsion huile-dans-l'eau protectrice de l'épiderme humain

| | | |
|---|---|---|
| - Composé de l'exemple 4 | | 2,0 g |
| - Alcool cétylstéarylique oxyéthyléné (C16/C18 - 35/65) (15 OE) ("MERGITAL CS 15" vendu par la Société HENKEL) | | 3,0 g |
| - Monostéarate de glycérol | | 4,8 g |
| - Alcool myristique | | 4,5 g |
| - Benzoate d'alcools C12/C15 ("FINSOLV TN" vendu par la Société WITCO) | | 18,0 g |
| - Propylène glycol | | 6,0 g |
| - Conservateur | | 0,2 g |
| - Parfum | | 0,6 g |
| - Eau déminéralisée | qsp | 100 g |

On chauffe les corps gras et les émulsionnants vers 80-85°C; on ajoute le composé de l'exemple 4. Par ailleurs, on chauffe à 80-85°C l'eau contenant les composés hydrosolubles et on ajoute la phase grasse à la phase aqueuse. Après 10 minutes d'agitation vive, on laisse refroidir sous agitation modérée, puis on ajoute le parfum et le conservateur.

Exemple B : Emulsion huile-dans-l'eau antisolaire

Elle est similaire à celle de l'exemple A, mais contient 2,0 g de composé de l'exemple 6 à la place du composé de l'exemple 4.

Exemple C : Emulsion huile-dans-l'eau antisolaire

| | | |
|---|---|---|
| - Composé de l'exemple 3 | | 2,0 g |
| - 2-hydroxy 4-méthoxy-benzophénone | | 1,0 g |
| - Lanoline liquide | | 7,0 g |
| - Triglycérides d'acides myristique/palmitique/ stéarique ("NESATOL" vendu par la Société VEVY) | | 5,0 g |
| - Triglycérides oléiques oxyéthylénés ("LUBRAFIL M1969 CS" vendu par la Société GATTEFOSSE) | | 2,5 g |
| - Mélange de monostéarate de glycérol et de stéarate de polyéthylène glycol (100 OE) ("ARLACEL 165" vendu par la Société SEPPIC) | | 5,0 g |
| - Alcool stéarylique | | 1,0 g |
| - Acide stéarique | | 2,5 g |
| - Mélange de phosphates de cétyle et de monocétyl phosphate de diéthanolamine "AMPHISOL NP" Société GIVAUDAN | | 0,5 g |
| - Benzoate d'alcools C12-C15 ("FINSOLV TN" vendu par la Société WITCO) | | 9,0 g |
| - Triéthanolamine | | 0,2 g |
| - Conservateur | | 0,4 g |
| - Parfum | | 0,6 g |
| - Eau déminéralisée | qsp | 100 g |

Cette émulsion est préparée de la même façon que dans l'exemple A.

Exemple D : Emulsion huile-dans-l'eau antisolaire

| | | |
|---|---|---|
| - Composé de l'exemple 1 | | 3,5 g |
| - Mélange d'alcools cétylstéarylique et cétyl stéarylique oxyéthyléné à 33 moles d'OE ("SINNOWAX AO" vendu par la Société HENKEL) | | 7,0 g |
| - Monostéarate de glycérol | | 2,0 g |
| - Alcool cétylique | | 1,3 g |
| - Propylène glycol | | 10,0 g |
| - Conservateur | | 0,2 g |
| - Parfum | | 0,6 g |
| - Benzoate d'alcools C12/C15 ("FINSOLV TN" vendu par la Société WITCO) | | 15,0 g |
| - Eau déminéralisée | qsp | 100 g |

Cette émulsion est préparée de la même façon que dans l'exemple A.

Exemple E : Stick antisolaire

| | | |
|---|---|---|
| - Composé de l'exemple 4 | | 5,0 g |
| - Cire minérale d'hydrocarbures | | 20,0 g |
| - Cire d'abeilles | | 7,0 g |
| - Alcool oléique | | 12,0 g |
| - Lanoline hydrogénée | | 8,0 g |
| - Lanoline liquide | | 8,0 g |
| - Cire de carnauba | | 1,0 g |
| - Benzoate d'alcools C12/C15 ("FINSOLV TN" vendu par la Société WITCO) | | 20,0 g |
| - Parfum | | 1,2 g |
| - Huile de vaseline | qsp | 100 g |

Exemple F : stick antisolaire

Il est similaire à celui de l'exemple E où l'on utilise 5,0 g de composé de l'exemple 9 à la place du composé de l'exemple 4.

Exemple G : Huile antisolaire

| | | |
|---|---|---|
| - Composé de l'exemple 3 | | 3,5 g |
| - Huile d'amandes douces | | 3,0 g |
| - Parfum | | 1,2 g |
| - Benzoate d'alcools C12/C15 ("FINSOLV TN" vendu par la Société WITCO) | qsp | 100 g |

Exemple H : Huile antisolaire

Elle est similaire à celle de l'exemple G où l'on remplace le composé de l'exemple 3 par le composé de l'exemple 8.

Exemple I : Emulsion eau-dans-l'huile antisolaire

| | | |
|---|---|---|
| - Composé de l'exemple 4 | | 3,0 g |
| - Mélange de diorgano polysiloxane à greffons cétyle et polyéthoxy-polypropoxy-propyloxy, d'oléate de polyglycérol et de laurate d'hexyle dénommé dans le dictionnaire CTFA cétyldiméthicone copolyol/cétyldiméthicone/polyglycérol 3-oléate/hexyl laurate ("ABIL WS 08" vendu par la Société GOLDSCHMIDT) | | 5,0 g |
| - Benzoate d'alcools C12/C15 ("FINSOLV TN" vendu par la Société WITCO) | | 12,0 g |
| - Vaseline | | 2,0 g |
| - Cire d'abeilles | | 2,5 g |
| - Glycérine | | 2,0 g |
| - Chlorure de sodium | | 2,0 g |
| - Conservateur | | 0,2 g |
| - Parfum | | 0,6 g |
| - Eau déminéralisée | qsp | 100 g |

Cette émulsion est préparée de la même façon que dans l'exemple A sauf que l'on ajoute la phase aqueuse à la phase grasse.

Exemple J : Emulsion huile-dans-l'eau antisolaire

| | | |
|---|---|---|
| - Composé de l'exemple 5 | | 1,7 g |
| - Composé de l'exemple 7 | | 1,8 g |
| - Mélange d'alcools cétylstéarylique et cétylstéarylique oxyéthyléné à 33 moles d'OE ("SINNOWAX AO" vendu par la Société HENKEL) | | 7 g |
| - Monostéarate de glycérol | | 2 g |
| - Alcool cétylique | | 1,3 g |
| - Propylène-glycol | | 10 g |
| - Benzoate d'alcools $C_{12}/C_{15}$ ("FINSOLV TN" vendu par la Société WITCO) | | 15 g |
| - Conservateur | | 0,2 g |
| - Eau déminéralisée | qsp | 100 g |

Cette émulsion est préparée de la même façon que dans l'exemple A.

Exemple K : Emulsion huile-dans-l'eau antisolaire

| | | |
|---|---|---|
| - Composé de l'exemple 2 | | 5,0 g |
| - Mélange d'alcool cétylstéarylique et cétylstéarylique oxyéthyléné à 33 moles d'OE ("SINNOWAX AO" vendu par la Société HENKEL) | | 7,0 g |
| - Monostéarate de glycérol | | 2,0 g |
| - Propylène glycol | | 10,0 g |
| - Alcool cétylique | | 1,3 g |
| - Benzoate d'alcools C12/C15 ("FINSOLV TN" vendu par la Société WITCO) | | 15 g |
| - Conservateur | | 0,2 g |
| - Eau déminéralisée | qsp | 100 g |

Cette émulsion est préparée de la même façon que dans l'exemple A.

Les compositions des exemples A à K ci-dessus sont destinées à l'application sur la peau pour la protéger du rayonnement UV.

Exemple L : Huile de soin protectrice des cheveux

| | |
|---|---|
| - Composé de l'exemple 1 | 1,0 g |
| - Alcool éthylique absolu | 50 g |
| - Tétraméthyl-1,3,3,5 tétraphényl-1,1,5,5 trisiloxane vendu sous le nom d'"Huile 763" par la Société RHONE POULENC | 65 g |

Ce produit se présente sous forme d'une huile limpide.

Appliquée sur cheveux séchés, cette huile facilite leur lissage et les rend brillants tout en les protégeant du rayonnement ultraviolet.

## Revendications

1. Utilisation en cosmétique de diorganopolysiloxanes à fonction benzalmalonate choisis parmi ceux de formule :

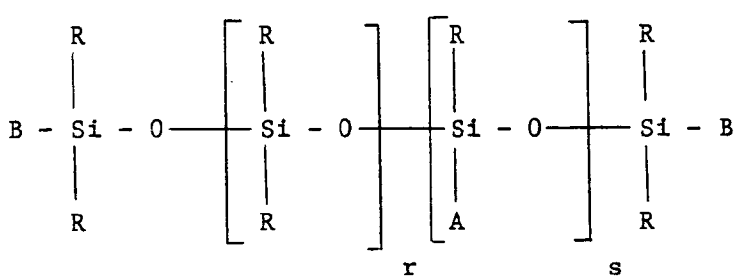

(1)

dans laquelle les symboles :

R, identiques ou différents, sont choisis parmi les radicaux alkyle en $C_1$- $C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A, et ceux de formule :

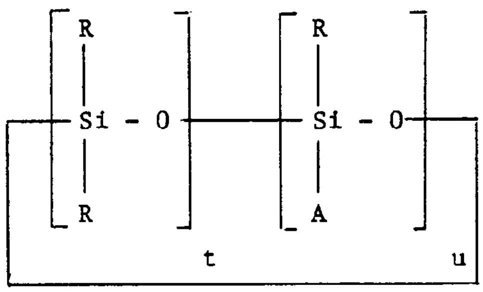

(2)

dans laquelle

R a la même signification qu'à la formule (1),

u est un nombre compris entre 1 et 20 inclus,

t est un nombre compris entre 0 et 20 inclus,

t + u est égal ou supérieur à 3,

formules dans lesquelles le symbole A est un radical de formule :

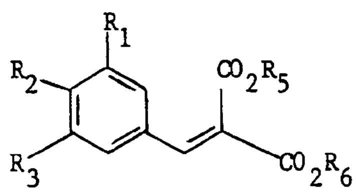

(3)

dans laquelle :

$R_1$ et $R_2$ sont choisis parmi un atome d'hydrogène, un radical hydroxyle, un radical triméthylsiloxy, un radical alkyle en $C_1$-$C_6$, un radical alcoxy en $C_1$-$C_6$, et un groupement divalent Y de formule :

$$-(O)_n-(CH_2)_p-\underset{R_4}{\underset{|}{CH}}-CH_2- \ ,$$

dans laquelle :

n est 0 ou 1,

p est un nombre entier compris entre 1 et 10 inclus, de préférence entre 1 et 4, et $R_4$ est choisi parmi un atome d'hydrogène et un radical alkyle en $C_1$-$C_4$, l'un des deux radicaux $R_1$ et $R_2$ représentant nécessairement le groupement Y,

$R_3$ représente un atome d'hydrogène, un radical alkyle en $C_1$-$C_6$ ou un radical alcoxy en $C_1$-$C_6$.

$R_5$ et $R_6$ identiques ou différents, représentent un radical alkyle en $C_1$-$C_8$.

2. Utilisation en cosmétique d'un polymère statistique ou à blocs de formule (1) ou (2) selon la revendication 1, présentant au moins l'une des caractéristiques suivantes :
   - R est méthyle,
   - B est méthyle,
   - $R_1$ est H ou Y,
   - $R_2$ est Y, méthoxy ou butoxy
   - $R_3$ est H ou méthoxy
   - n = 0 ou 1
   - p = 1
   - $R_4$ est H ou méthyle

- $R_5$ et $R_5$ sont éthyle ou 2-éthylhexyle
- r est compris entre 5 et 20 inclus
- s est compris entre 2 et 15 inclus
- t + u est compris entre 3 et 10 inclus.

**3.** Utilisation en cosmétique à titre d'agents filtrant le rayonnement UV de longueurs d'onde comprises entre 280 et 360 nm, de diorganopolysiloxanes à fonction benzalmalonate choisis parmi ceux de formule :

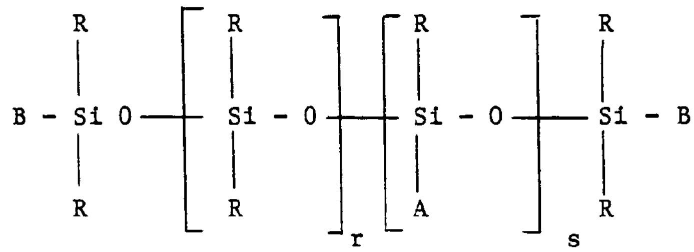

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

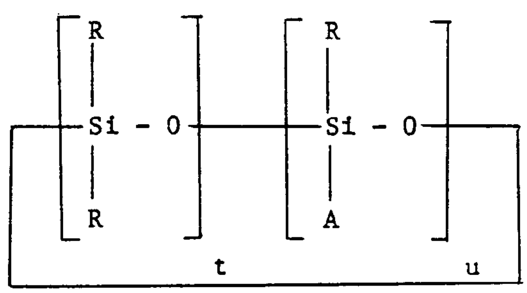

(2)

dans laquelle

R a la même signification qu'à la formule (1),

u est un nombre compris entre 1 et 20 inclus,

t est un nombre compris entre 0 et 20 inclus,

t + u est égal ou supérieur à 3,

formules dans lesquelles le symbole A est un radical de formule :

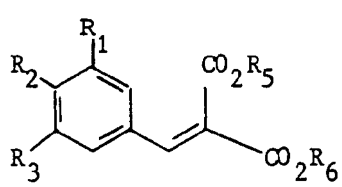

(3)

dans laquelle :

$R_1$ et $R_2$ sont choisis parmi un atome d'hydrogène, un radical hydroxyle, un radical triméthylsiloxy, un radical alkyle en $C_1$-$C_6$, un radical alcoxy en $C_1$-$C_6$, et un groupement divalent Y de formule :

$$-(O)_n-(CH_2)_p-\underset{\displaystyle R_4}{CH}-CH_2- \ ,$$

dans laquelle :

- n est 0 ou 1,
- p est un nombre entier compris entre 1 et 10 inclus, de préférence entre 1 et 4, et $R_4$ est choisi parmi un atome d'hydrogène et un radical alkyle en $C_1$-$C_4$, l'un des deux radicaux $R_1$ et $R_2$ représentant nécessairement le groupement Y,

  $R_3$ représente un atome d'hydrogène ou un radical alkyle en $C_1$-$C_6$ ou un radical alcoxy en $C_1$-$C_6$, $R_5$ et $R_6$, identiques ou différents, représentent un radical alkyle en $C_1$-$C_8$.

**4.** Utilisation en cosmétique à titre d'agents filtrant le rayonnement UV de longueurs d'onde comprises entre 280 et 360 nm, de diorganopolysiloxanes statistiques ou à blocs de formule (1) ou (2) selon la revendication 3, présentant au moins l'une des caractéristiques suivantes :

- R est méthyle
- B est méthyle
- $R_1$ est H ou Y
- $R_2$ est Y, méthoxy ou butoxy
- $R_3$ est H ou méthoxy
- n = 0 ou 1
- p = 1
- $R_4$ est H ou méthyle
- $R_6$ et $R_6$ sont éthyle ou 2-éthylhexyle
- r est compris entre 5 et 20 inclus,
- s est compris entre 2 et 15 inclus,
- t + u est compris entre 3 et 10 inclus,

**5.** Utilisation en cosmétique à titre d'agent filtrant le rayonnement UV de longueurs d'onde comprises entre 280 et 360 nm, d'un polydiméthylsiloxane à fonction benzalmalonate de formule (1) selon la revendication 1 dans laquelle R et B désignent méthyle, r = 5, s =5, $R_1$ est H, $R_2$ est Y avec n = 1, p = 1 et $R_4$ est H, $R_3$ est H ou méthoxy et $R_6$ et $R_6$ sont éthyle.

**6.** Utilisation en cosmétique à titre d'agent filtrant le rayonnement UV de longueurs d'onde comprises entre 280 et 360 nm d'un polydiméthylsiloxane à fonction benzalmalonate de formule (1) selon la revendication 1, dans laquelle R et B désignent méthyle r = 20, s = 5, $R_1$ est H, $R_2$ est Y avec n = 1, p = 1 et $R_4$ est H, $R_3$ est H et $R_6$ et $R_6$ sont éthyle.

**7.** Utilisation en cosmétique à titre d'agent filtrant le rayonnement UV de longueurs d'onde comprises entre 280 et 360 nm d'un polydiméthylsiloxane à fonction benzalmalonate de formule (1) selon la revendication 1 dans laquelle R et B désignent méthyle, r = 0, s = 40, $R_1$ est H, $R_2$ est Y avec n = 1, p =1 et $R_4$ est H, $R_3$ est H et $R_6$ et $R_6$ sont éthyle.

**8.** Utilisation en cosmétique à titre d'agent filtrant le rayonnement UV de longueurs d'onde comprises entre 280 et 360 nm d'un polydiméthylsiloxane à fonction benzalmalonate de formule (1) selon la revendication 1 dans laquelle R et B désignent méthyle, r = 7,3, s = 9, $R_1$ est H, $R_2$ est Y avec n = 1, p = 1 et $R_4$ est $CH_3$, $R_3$ est H et $R_6$ et $R_6$ sont éthyle.

**9.** Utilisation en cosmétique à titre d'agent filtrant le rayonnement UV de longueurs d'onde comprises entre 280 et 360 nm d'un polydiméthylsiloxane à fonction benzalmalonate de formule (1) selon la revendication 1 dans laquelle R et B désignent méthyle, r = 5, s = 5, $R_1$ est Y avec n = 0, p = 1 et $R_4$ est H, $R_2$ est méthoxy, $R_3$ est H ou méthoxy et $R_5$ et $R_6$ sont éthyle.

**10.** Utilisation en cosmétique à titre d'agent filtrant le rayonnement UV de longueurs d'onde comprises entre 280 et 360 nm d'un polydiméthylsiloxane à fonction benzalmalonate de formule (1) selon la revendication 1 dans laquelle R et B désignent méthyle, r = 5, s = 5, $R_1$ est Y avec n = 0, p = 1 et $R_4$ est H, $R_2$ est méthoxy, $R_3$ est méthoxy et $R_6$ et $R_6$ sont 2-éthylhexyle.

**11.** Utilisation en cosmétique à titre d'agent filtrant le rayonnement UV de longueurs d'onde comprises entre 280 et 360 nm d'un polydiméthylsiloxane à fonction benzalmalonate de formule (1) selon la revendication 1 dans laquelle R et B désignent méthyle, r = 5, s = 5, $R_1$ est Y avec n = 0, p = 1 et $R_4$ est H, $R_2$ est butoxy, $R_3$ est méthoxy et $R_5$ et $R_6$ sont éthyle.

**12.** Composition cosmétique, caractérisée par le fait qu'elle comprend, dans un support cosmétiquement acceptable, une quantité efficace d'au moins un diorganopolysiloxane à fonction benzalmalonate choisi parmi ceux de formule :

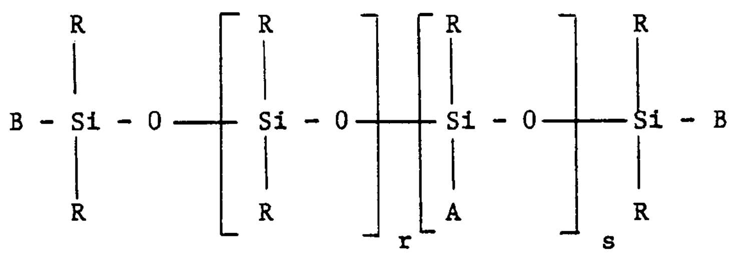

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

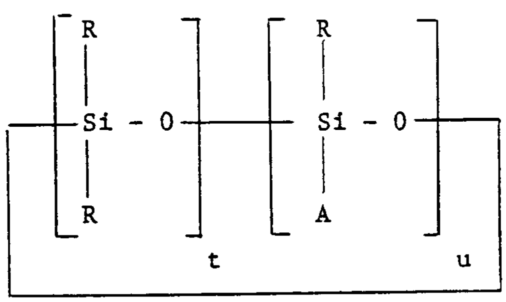

(2)

dans laquelle

R a la même signification qu'à la formule (1),

u est un nombre compris entre 1 et 20 inclus,

t est un nombre compris entre 0 et 20 inclus,

t + u est égal ou supérieur à 3,

formules dans lesquelles le symbole A est un radical de formule :

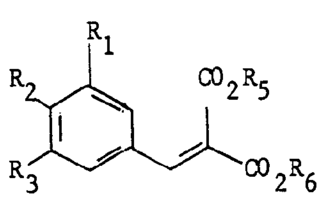

(3)

dans laquelle :

$R_1$ et $R_2$ sont choisis parmi un atome d'hydrogène, un radical hydroxyle, un radical triméthylsiloxy, un radical alkyle en $C_1$-$C_6$, un radical alcoxy en $C_1$-$C_6$, et un groupement divalent Y de formule :

$$-(O)_n-(CH_2)_p-\underset{R_4}{CH}-CH_2- ,$$

dans laquelle :

- n est 0 ou 1,
- p est un nombre entier compris entre 1 et 10 inclus et $R_4$ est choisi parmi un atome d'hydrogène et un radical alkyle en $C_1$-$C_4$, l'un des deux radicaux $R_1$ et $R_2$ représentant nécessairement le groupement Y,
- $R_3$ représente un atome d'hydrogène, un radical alkyle en $C_1$-$C_6$ ou un radical alcoxy en $C_1$-$C_6$,
- $R_5$ et $R_5$, identiques ou différents, représentent un radical alkyle en $C_1$-$C_8$.

**13.** Composition cosmétique selon la revendication 12, caractérisée par le fait qu'elle comprend un diorganopolysiloxane à fonction benzalmalonate de formule (1) ou (2), statistique ou à blocs, présentant au moins l'une des caractéristiques suivantes :

- R est méthyle
- B est méthyle
- $R_1$ est H ou Y
- $R_2$ est Y, méthoxy ou butoxy
- $R_3$ est H ou méthoxy
- n est 0 ou 1
- p = 1
- $R_4$ est H ou méthyle
- $R_5$ et $R_5$ sont éthyle ou 2-éthylhexyle
- r est compris entre 5 et 20 inclus
- s est compris entre 2 et 15 inclus
- t + u est compris entre 3 et 10 inclus.

**14.** Composition cosmétique selon la revendication 12 ou 13, caractérisée par le fait qu'elle comprend un polydiméthylsiloxane à fonction benzalmalonate de formule (1) selon la revendication 1 dans laquelle R et B désignent méthyle, r = 5, s = 5, $R_1$ est H, $R_2$ est Y avec n = 1, p = 1 et $R_4$ est H, $R_3$ est H ou méthoxy et $R_5$ et $R_6$ sont éthyle.

**15.** Composition cosmétique selon la revendication 12 ou 13, caractérisée par le fait qu'elle comprend un polydiméthylsiloxane à fonction benzalmalonate de formule (1) selon la revendication 1 dans laquelle R et B désignent méthyle, r = 20, s = 5, $R_1$ est H, $R_2$ est Y avec n = 1, p = 1 et $R_4$ est H, $R_3$ est H et $R_5$ et $R_5$ sont éthyle.

**16.** Composition cosmétique selon la revendication 12 ou 13, caractérisée par le fait qu'elle comprend un polydiméthylsiloxane à fonction benzalmalonate de formule (1) selon la revendication 1 dans laquelle R et B désignent méthyle, r = 0, s = 40, $R_1$ est H, $R_2$ est Y avec n = 1, p = 1 et $R_4$ est H, $R_3$ est H et $R_5$ et $R_5$ sont éthyle.

**17.** Composition cosmétique selon la revendication 12 ou 13, caractérisée par le fait qu'elle comprend un poydiméthylsiloxane à fonction benzalmalonate de formule (1) selon la revendication 1 dans laquelle R et B désignent méthyle, r = 7,3, s = 9, $R_1$ est H, $R_2$ est Y avec n = 1, p = 1 et $R_4$ est $CH_3$, $R_3$ est H et $R_5$ et $R_5$ sont éthyle.

**18.** Composition cosmétique selon la revendication 12 ou 13, caractérisée par le fait qu'elle comprend un polydiméthylsiloxane à fonction benzalmalonate de formule (1) selon la revendication 1 dans laquelle R et B désignent méthyle, r = 5, s = 5, $R_1$ est Y avec n = 0, p = 1 et $R_4$ est H, $R_2$ est méthoxy, $R_3$ est H ou méthoxy et $R_5$ et $R_5$ sont éthyle.

**19.** Composition cosmétique selon la revendication 12 ou 13, caractérisée par le fait qu'elle comprend un polydiméthylsiloxane à fonction benzalmalonate de formule (1) selon la revendication 1 dans laquelle R et B désignent méthyle, r = 5, s = 5, $R_1$ est Y avec n = 0, p = 1 et $R_4$ est H, $R_2$ est méthoxy, $R_3$ est méthoxy et $R_5$ et $R_6$ sont 2-éthylhexyle.

**20.** Composition cosmétique selon la revendication 12 ou 13, caractérisée par le fait qu'elle comprend un polydiméthylsiloxane à fonction benzalmalonate de formule (1) selon la revendication 1 dans laquelle R et B désignent méthyle, r = 5, s = 5, $R_1$ est Y avec n = 0, p = 1 et $R_4$ est H, $R_2$ est butoxy, $R_3$ est méthoxy et $R_5$ et $R_6$ sont éthyle.

**21.** Composition cosmétique selon l'une quelconque des revendications 12 à 20, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, benzoates d'alcools en $C_{12}$-$C_{15}$, propulseurs, colorants et pigments.

**22.** Composition cosmétique selon l'une quelconque des revendications 12 à 21, caractérisée par le fait qu'elle se présente sous forme de lotion huileuse, alcoolique ou oléoalcoolique, d'émulsion, gel oléoalcoolique, alcoolique ou hydroalcoolique, bâtonnet solide, spray ou aérosol.

**23.** Composition cosmétique selon l'une quelconque des revendications 12 à 22, caractérisée par le fait qu'elle constitue une composition protectrice de l'épiderme humain et contient 0,25 à 3% en poids de diorganopolysiloxane de formule (1) ou (2).

**24.** Composition cosmétique selon l'une quelconque des revendications 12 à 22, se présentant sous forme de composition anti-solaire, caractérisée par le fait qu'elle contient 0,5 à 15% en poids de diorganopolysiloxane de formule (1) ou (2).

**25.** Composition cosmétique anti-solaire selon la revendication 24, caractérisée par le fait qu'elle contient en outre un agent filtrant les rayons UV-B et/ou UV-A.

**26.** Composition cosmétique selon l'une quelconque des revendications 12 à 21, destinée à être appliquée sur les cheveux, caractérisée par le fait qu'elle se présente sous forme de shampooing, lotion, gel ou émulsion à rincer, à appliquer avant ou après le shampooing, avant ou après coloration ou décoloration, avant ou après permanente, lotion ou gel coiffants ou traitants, lotion ou gel pour le brushing ou la mise en plis, spray de coiffage, laque pour cheveux, et comprend 0,25 à 5% en poids de diorganopolysiloxane de formule (1) ou (2).

**27.** Composition cosmétique selon l'une quelconque des revendications 12 à 21, se présentant sous forme d'une composition cosmétique colorée ou non, caractérisée par le fait qu'elle est constituée par une composition capillaire, un produit de maquillage ou une composition pour les soins ou le traitement de la peau, comprenant 0,25 à 3% en poids de diorganopolysiloxane de formule (1) ou (2).

**28.** Procédé cosmétique de protection de la peau et des cheveux naturels ou sensibilisés contre le rayonnement ultraviolet, caractérisé par le fait qu'il consiste à appliquer sur la peau ou les cheveux une quantité efficace d'une composition cosmétique contenant au moins un diorganopolysiloxane à fonction benzalmalonate de formule (1) ou (2) défini dans l'une quelconque des revendications 3 à 11.

**29.** Procédé de protection d'une composition cosmétique contre les rayons ultraviolets, caractérisé par le fait qu'il consiste à incorporer à cette composition une quantité efficace d'au moins un diorganopolysiloxane à fonction benzalmalonate de formule (1) ou (2) définie dans l'une quelconque des revendications 3 à 11.

## Claims

**1.** Use in cosmetics of diorganopolysiloxanes containing a benzalmalonate functional group chosen from those of formula:

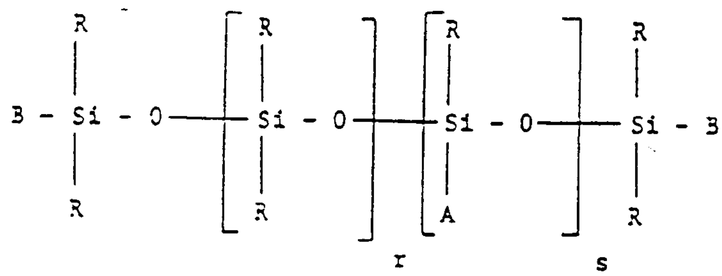

(1)

in which the symbols:

R, which are identical or different, are chosen from $C_1$-$C_{10}$ alkyl, phenyl and 3,3,3-trifluoropropyl radicals, at least 80% in numerical terms of the radicals R being methyl radicals,

B, which are identical or different, are chosen from the radicals R and the radical A,

r is a number chosen between 0 and 200 inclusive,

s is a number chosen between 0 and 50 inclusive and if s is 0, at least one of the two symbols B denotes A,

and those of formula:

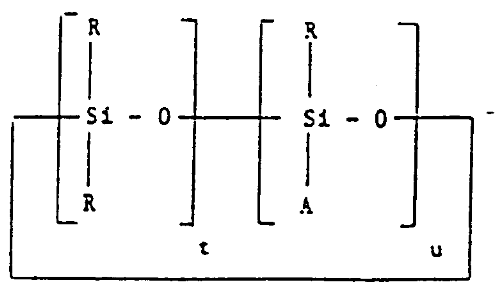

(2)

in which

R has the same meaning as in the formula (1),

u is a number between 1 and 20 inclusive,

t is a number between 0 and 20 inclusive,

t + u is not less than 3,

formulae in which the symbol A is a radical of formula:

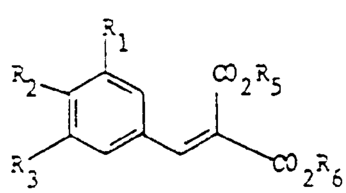

(3)

in which:

$R_1$ and $R_2$ are chosen from a hydrogen atom, a hydroxyl radical, a trimethylsiloxy radical, a $C_1$-$C_6$ alkyl radical, a $C_1$-$C_6$ alkoxy radical, and a divalent group Y of formula:

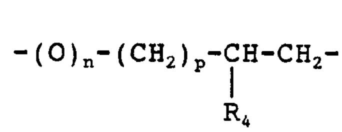

in which:

n is 0 or 1,

p is an integer between 1 and 10 inclusive, preferably between 1 and 4, and $R_4$ is chosen from a hydrogen atom and a $C_1$-$C_4$ alkyl radical, one of the two radicals $R_1$ and $R_2$ necessarily representing the

group Y,

$R_3$ represents a hydrogen atom, a $C_1$-$C_6$ alkyl radical or a $C_1$-$C_6$ alkoxy radical,

$R_5$ and $R_6$, which are identical or different, represent a $C_1$-$C_8$ alkyl radical.

**2.** Use in cosmetics of a random or block polymer of formula (1) or (2) according to Claim 1, having at least one of the following characteristics:

- R is methyl,
- B is methyl,
- $R_1$ is H or Y,
- $R_2$ is Y, methoxy or butoxy
- $R_3$ is H or methoxy
- n = 0 or 1
- p = 1
- $R_4$ is H or methyl
- $R_5$ and $R_5$ are ethyl or 2-ethylhexyl
- r is between 5 and 20 inclusive
- s is between 2 and 15 inclusive
- t + u is between 3 and 10 inclusive.

**3.** Use in cosmetics as agents screening out UV radiation of wavelengths between 280 and 360 nm, of diorganopolysiloxanes containing a benzalmalonate functional group chosen from those of formula:

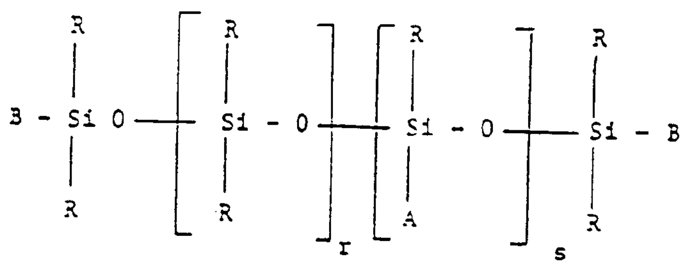

(1)

in which the symbols:

R, which are identical or different, are chosen from $C_1$-$C_{10}$ alkyl, phenyl and 3,3,3-trifluoropropyl radicals, at least 80% in numerical terms of the radicals R being methyl radicals,

B, which are identical or different, are chosen from the radicals R and the radical A,

r is a number chosen between 0 and 200 inclusive,

s is a number chosen between 0 and 50 inclusive and if s is 0, at least one of the two symbols B denotes A,

and those of formula:

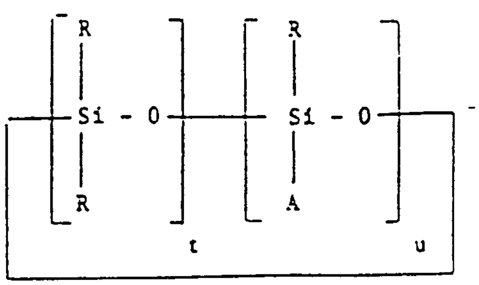

(2)

in which

R has the same meaning as in the formula (1),

u is a number between 1 and 20 inclusive,

t is a number between 0 and 20 inclusive,

t + u is not less than 3,

formulae in which the symbol A is a radical of formula:

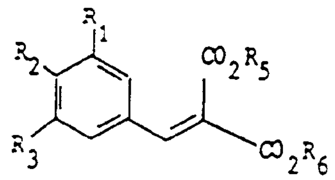

(3)

in which:

$R_1$ and $R_2$ are chosen from a hydrogen atom, a hydroxyl radical, a trimethylsiloxy radical, a $C_1$-$C_6$ alkyl radical, a $C_1$-$C_6$ alkoxy radical, and a divalent group Y of formula:

$$-(O)_n-(CH_2)_p-\underset{\displaystyle R_4}{\underset{|}{CH}}-CH_2-$$

in which:

- n is 0 or 1,
- p is an integer between 1 and 10 inclusive, preferably between 1 and 4, and $R_4$ is chosen from a hydrogen atom and a $C_1$-$C_4$ alkyl radical, one of the two radicals $R_1$ and $R_2$ necessarily representing the group Y,

$R_3$ represents a hydrogen atom, a $C_1$-$C_6$ alkyl radical or a $C_1$-$C_6$ alkoxy radical,

$R_5$ and $R_6$, which are identical or different, represent a $C_1$-$C_8$ alkyl radical.

4. Use in cosmetics as agents screening out UV radiation of wavelengths between 280 and 360 nm, of random or block diorganopolysiloxanes of formula (1) or (2) according to Claim 3, having at least one of the following characteristics:
   - R is methyl
   - B is methyl
   - $R_1$ is H or Y
   - $R_2$ is Y, methoxy or butoxy
   - $R_3$ is H or methoxy
   - n = 0 or 1
   - p = 1
   - $R_4$ is H or methyl
   - $R_5$ and $R_5$ are ethyl or 2-ethylhexyl
   - r is between 5 and 20 inclusive,
   - s is between 2 and 15 inclusive,
   - t + u is between 3 and 10 inclusive.

5. Use in cosmetics as an agent screening out UV radiation of wavelengths between 280 and 360 nm, of a polydimethylsiloxane containing a benzalmalonate functional group of formula (1) according to Claim 1 in which R and B denote methyl, r = 5, s = 5, $R_1$ is H, $R_2$ is Y with n = 1, p = 1 and $R_4$ is H, $R_3$ is H or methoxy and $R_5$ and $R_5$ are ethyl.

6. Use in cosmetics as an agent screening out UV radiation of wavelengths between 280 and 360 nm of a polydimethylsiloxane containing a benzalmalonate functional group of formula (1) according to Claim 1, in which R and B denote methyl, r = 20, s = 5, $R_1$ is H, $R_2$ is Y with n = 1, p = 1 and $R_4$ is H, $R_3$ is H and $R_5$ and $R_6$ are ethyl.

7. Use in cosmetics as an agent screening out UV radiation of wavelengths between 280 and 360 nm of a polydimethylsiloxane containing a benzalmalonate functional group of formula (1) according to Claim 1 in which R and B denote methyl, r = 0, s = 40, $R_1$ is H, $R_2$ is Y with n = 1, p = 1 and $R_4$ is H, $R_3$ is H and $R_5$ and $R_5$ are ethyl.

8. Use in cosmetics as an agent screening out UV radiation of wavelengths between 280 and 360 nm of a polydimethylsiloxane containing a benzalmalonate functional group of formula (1) according to Claim 1 in which R and B denote methyl, r = 7.3, s = 9, $R_1$ is H, $R_2$ is Y with n = 1, p = 1 and $R_4$ is $CH_3$, $R_3$ is H and

$R_5$ and $R_6$ are ethyl.

**9.** Use in cosmetics as an agent screening out UV radiation of wavelengths between 280 and 360 nm of a polydimethylsiloxane containing a benzalmalonate functional group of formula (1) according to Claim 1 in which R and B denote methyl, r = 5, s = 5, $R_1$ is Y with n = 0, p = 1 and $R_4$ is H, $R_2$ is methoxy, $R_3$ is H or methoxy and $R_5$ and $R_6$ are ethyl.

**10.** Use in cosmetics as an agent screening out UV radiation of wavelengths between 280 and 360 nm of a polydimethylsiloxane containing a benzalmalonate functional group of formula (1) according to Claim 1 in which R and B denote methyl, r = 5, s = 5, $R_1$ is Y with n = 0, p = 1 and $R_4$ is H, $R_5$ is methoxy, $R_3$ is methoxy and $R_5$ and $R_5$ are 2-ethylhexyl.

**11.** Use in cosmetics as an agent screening out UV radiation of wavelengths between 280 and 360 nm of a polydimethylsiloxane containing a benzalmalonate functional group of formula (1) according to Claim 1 in which R and B denote methyl, r = 5, s = 5, $R_1$ is Y with n = 0, p = 1 and $R_4$ is H, $R_2$ is butoxy, $R_3$ is methoxy and $R_5$ and $R_5$ are ethyl.

**12.** Cosmetic composition, characterised in that it comprises, in a cosmetically acceptable carrier, an effective amount of at least one diorganopolysiloxane containing a benzalmalonate functional group chosen from those of formula:

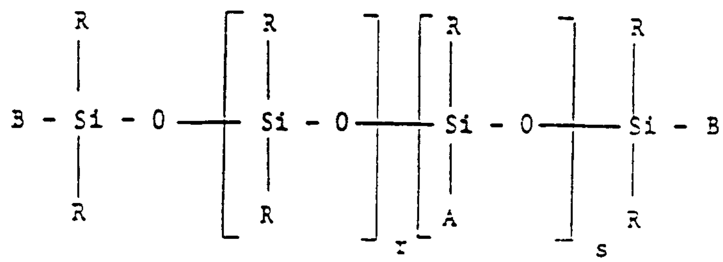

(1)

in which the symbols:

R, which are identical or different, are chosen from $C_1$-$C_{10}$ alkyl, phenyl and 3,3,3-trifluoropropyl radicals, at least 80% in numerical terms of the radicals R being methyl radicals,

B, which are identical or different, are chosen from the radicals R and the radical A,

r is a number chosen between 0 and 200 inclusive,

s is a number chosen between 0 and 50 inclusive and if s is 0, at least one of the two symbols B denotes A,

and those of formula:

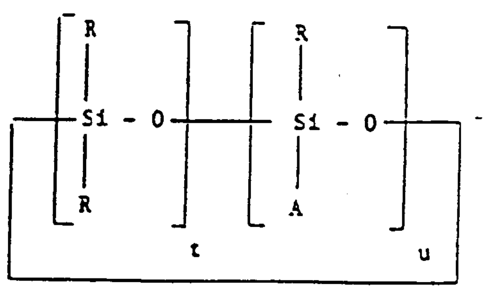

(2)

in which

R has the same meaning as in the formula (1),

u is a number between 1 and 20 inclusive,

t is a number between 0 and 20 inclusive,

t + u is not less than 3,

formulae in which the symbol A is a radical of formula:

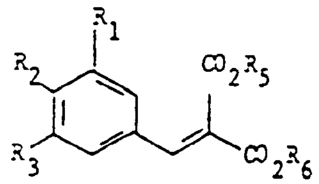

(3)

in which:

$R_1$ and $R_2$ are chosen from a hydrogen atom, a hydroxyl radical, a trimethylsiloxy radical, a $C_1$-$C_6$ alkyl radical, a $C_1$-$C_6$ alkoxy radical, and a divalent group Y of formula:

$$-(O)_n-(CH_2)_p-\underset{\displaystyle R_4}{\underset{|}{C}H}-CH_2-$$

in which:
- n is 0 or 1,
- p is an integer between 1 and 10 inclusive and $R_4$ is chosen from a hydrogen atom and a $C_1$-$C_4$ alkyl radical, one of the two radicals $R_5$ and $R_2$ necessarily representing the group Y,
- $R_3$ represents a hydrogen atom, a $C_1$-$C_6$ alkyl radical or a $C_1$-$C_6$ alkoxy radical,
- $R_5$ and $R_5$, which are identical or different, represent a $C_1$-$C_8$ alkyl radical.

**13.** Cosmetic composition according to Claim 12, characterised in that it comprises a random or block diorganopolysiloxane containing a benzalmalonate functional group of formula (1) or (2), having at least one of the following characteristics:
- R is methyl
- B is methyl
- $R_1$ is H or Y
- $R_2$ is Y, methoxy or butoxy
- $R_3$ is H or methoxy
- n = 0 or 1
- p =1
- $R_4$ is H or methyl
- $R_5$ and $R_5$ are ethyl or 2-ethylhexyl
- r is between 5 and 20 inclusive
- s is between 2 and 15 inclusive
- t + u is between 3 and 10 inclusive.

**14.** Cosmetic composition according to Claim 12 or 13, characterised in that it comprises a polydimethylsiloxane containing a benzalmalonate functional group of formula (1) according to Claim 1 in which R and B denote methyl, r = 5, s = 5, $R_1$ is H, $R_2$ is Y with n = 1, p = 1 and $R_4$ is H, $R_3$ is H or methoxy and $R_5$ and $R_6$ are ethyl.

**15.** Cosmetic composition according to Claim 12 or 13, characterised in that it comprises a polydimethylsiloxane containing a benzalmalonate functional group of formula (1) according to Claim 1 in which R and B denote methyl, r = 20, s = 5, $R_1$ is H, $R_2$ is Y with n = 1, p = 1 and $R_4$ is H, $R_3$ is H and $R_5$ and $R_6$ are ethyl.

**16.** Cosmetic composition according to Claim 12 or 13, characterised in that it comprises a polydimethylsiloxane containing a benzalmalonate functional group of formula (1) according to Claim 1 in which R and B denote methyl, r = 0, s = 40, $R_1$ is H, $R_2$ is Y with n = 1, p = 1 and $R_4$ is H, $R_3$ is H and $R_5$ and $R_6$ are ethyl.

**17.** Cosmetic composition according to Claim 12 or 13, characterised in that it comprises a polydimethylsiloxane containing a benzalmalonate functional group of formula (1) according to Claim 1 in which R and B denote methyl, r = 7.3, s = 9, $R_1$ is H, $R_2$ is Y with n = 1, p = 1 and $R_4$ is $CH_3$, $R_3$ is H and $R_5$ and $R_5$ are ethyl.

**18.** Cosmetic composition according to Claim 12 or 13, characterised in that it comprises a polydimethylsiloxane containing a benzalmalonate functional group of formula (1) according to Claim 1 in which R and B denote methyl, r = 5, s = 5, $R_1$ is Y with n = 0, p = 1 and $R_4$ is H, $R_2$ is methoxy, $R_3$ is H or methoxy and $R_5$ and $R_6$ are ethyl.

**19.** Cosmetic composition according to Claim 12 or 13, characterised in that it comprises a polydimethylsiloxane containing a benzalmalonate functional group of formula (1) according to Claim 1 in which R and B denote methyl, r = 5, s = 5, $R_1$ is Y with n = 0, p = 1 and $R_4$ is H, $R_2$ is methoxy, $R_3$ is methoxy and $R_5$ and $R_6$ are 2-ethylhexyl.

**20.** Cosmetic composition according to Claim 12 or 13, characterised in that it comprises a polydimethylsiloxane containing a benzalmalonate functional group of formula (1) according to Claim 1 in which R and B denote methyl, r = 5, s = 5, $R_1$ is Y with n = 0, p = 1 and $R_4$ is H, $R_2$ is butoxy, $R_3$ is methoxy and $R_5$ and $R_6$ are ethyl.

**21.** Cosmetic composition according to any one of Claims 12 to 20, characterised in that it contains, in addition, cosmetic adjuvants chosen from thickeners, demulcents, humectants, surface-active agents, preservatives, antifoams, perfumes, oils, waxes, lanolin, lower alcohols and polyols, $C_{12}$-$C_{15}$ alcohol benzoates, propellants, colourants and pigments.

**22.** Cosmetic composition according to any one of Claims 12 to 21, characterised in that it is provided in the form of an oily, alcoholic or oil-alcoholic lotion, an emulsion, an oil-alcoholic, alcoholic or dilute alcoholic gel, a solid stick, a spray or an aerosol.

**23.** Cosmetic composition according to any one of Claims 12 to 22, characterised in that it constitutes a composition which protects the human epidermis, and contains 0.25 to 3% by weight of a diorganopolysiloxane of formula (1) or (2).

**24.** Cosmetic composition according to any one of Claims 12 to 22, which is provided in the form of an antisun composition, characterised in that it contains 0.5 to 15% by weight of a diorganopolysiloxane of formula (1) or (2).

**25.** Antisun cosmetic composition according to Claim 24, characterised in that it contains, in addition, an agent screening out UV-B and/or UV-A rays.

**26.** Cosmetic composition according to any one of Claims 12 to 21, which is intended to be applied to the hair, characterised in that it is provided in the form of a shampoo, lotion, rinse-off gel or emulsion, to be applied before or after shampooing, before or after colouring or bleaching, before or after permanent waving, hair styling or treating lotion or gel, lotion or gel for blow drying or for hair setting, hair styling spray, hair lacquer, and comprises 0.25 to 5% by weight of a diorganopolysiloxane of formula (1) or (2).

**27.** Cosmetic composition according to any one of Claims 12 to 21, which is provided in the form of a coloured or colourless cosmetic composition, characterised in that it consists of a hair composition, a make up product or a composition for the care or treatment of the skin, comprising 0.25 to 3% by weight of a diorganopolysiloxane of formula (1) or (2).

**28.** Cosmetic process for protecting the skin and natural or sensitised hair from ultraviolet radiation, characterised in that it consists in applying to the skin or hair an effective amount of a cosmetic composition containing at least one diorganopolysiloxane containing a benzalmalonate functional group of formula (1) or (2) defined in any one of Claims 3 to 11.

**29.** Process for protecting a cosmetic composition against ultraviolet rays, characterised in that it consists in incorporating into this composition an effective amount of at least one diorganopolysiloxane containing a benzalmalonate functional group of formula (1) or (2) defined in any one of Claims 3 to 11.

## Patentansprüche

**1.** Verwendung von Diorganopolysiloxanen mit einer Benzalmalonatfunktion, die ausgewählt sind unter Verbindungen der Formel:

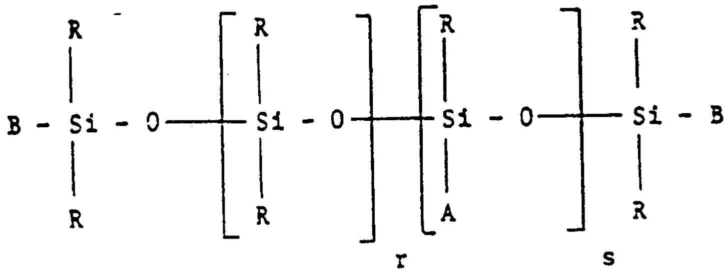 (1)

worin
die Reste R, die gleich oder verschieden sein können, ausgewählt sind unter $C_1$-$C_{10}$-Alkyl, Phenyl und 3,3,3-Trifluorpropyl, wobei wenigstens 80% der Reste R einen Methylrest bedeuten,
die Reste B, die gleich oder verschieden sein können, ausgewählt sind unter den Resten R und dem Rest A,
r eine Zahl zwischen 0 und 200 einschließlich ist,
s eine Zahl zwischen 0 und 50 einschließlich ist, und
wenn s für 0 steht, wenigstens einer der Reste B für A steht,
sowie unter Verbindungen der Formel:

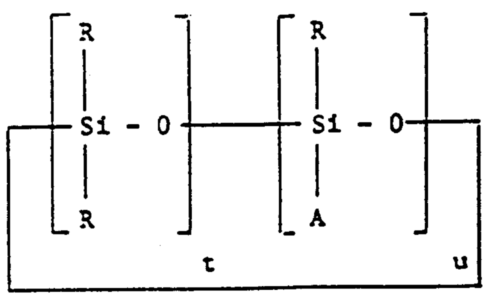 (2)

worin
R die gleichen Bedeutungen besitzt wie oben für die Formel (1) angegeben,
u eine Zahl zwischen 1 und 20 einschließlich bedeutet,
t eine Zahl zwischen 0 und 20 einschließlich bedeutet,
t + u gleich oder größer als 3 ist,
wobei in den obigen Formeln A für einen Rest der Formel:

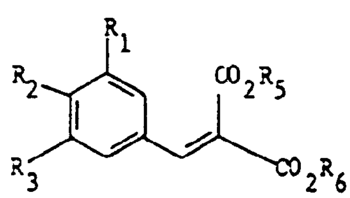 (3)

steht, worin $R_1$ und $R_2$ ausgewählt sind unter einem Wasstoffatom, einer Hydroxy-, Trimethylsilyloxy-, $C_1$-$C_6$-Alkyl-, $C_1$-$C_6$-Alkoxygruppe und einer divalenten Gruppe Y der Formel:

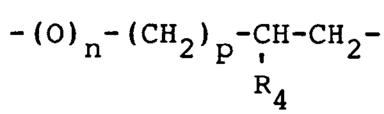

worin n für 0 oder 1 steht,
p eine ganze Zahl zwischen 1 und 10 einschließlich, vorzugsweise zwischen 1 und 4, bedeutet und $R_4$ ausgewählt ist unter einem Wasserstoffatom und einer $C_1$-$C_4$-Alkylgruppe, wobei einer der Reste $R_1$ und $R_2$ für die Gruppe Y stehen muß,

$R_3$ ein Wasserstoffatom, eine $C_1$-$C_6$-Alkyl- oder $C_1$-$C_6$-Alkoxygruppe bedeutet,
$R_5$ und $R_6$, die gleich oder verschieden sein können, eine $C_1$-$C_8$-Alklygruppe bedeutet,
in der Kosmetik.

2. Verwendung eines statistischen Polymers oder eines Blockpolymers der Formel (1) oder (2) nach Anspruch 1, die wenigstens eines der folgenden Charakteristika aufweisen:
R bedeutet Methyl,
B bedeutet Methyl,
$R_1$ bedeutet H oder Y,
$R_2$ bedeutet Y, Methoxy oder Butoxy,
$R_3$ bedeutet H oder Methoxy,
n = 0 oder 1,
p = 1,
$R_4$ bedeutet H oder Methyl,
$R_5$ und $R_6$ bedeuten Ethyl oder 2-Ethylhexyl,
r liegt zwischen 5 und 20 einschließlich,
s liegt zwischen 2 und 15 einschließlich,
t + u liegt zwischen 3 und 10 einschließlich, in der Kosmetik.

3. Verwendung von Diorganopolysiloxanen mit einer Benzalmalonatfunktion, die ausgewählt sind unter Verbindungen der Formel:

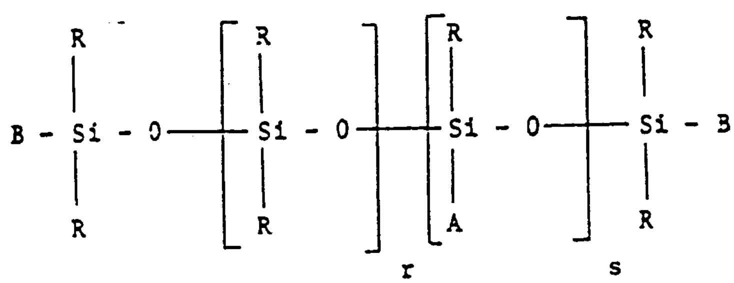

(1)

worin
die Reste R, die gleich oder verschieden sein können, ausgewählt sind unter $C_1$-$C_{10}$-Alkyl, Phenyl und 3,3,3-Trifluorpropyl, wobei wenigstens 80% der Reste R einen Methylrest bedeuten,
die Reste B, die gleich oder verschieden sein können, ausgewählt sind unter den Resten R und dem Rest A,
r eine Zahl zwischen 0 und 200 einschließlich ist,
s eine Zahl zwischen 0 und 50 einschließlich ist, und
wenn s für 0 steht, wenigstens einer der Reste B für A steht,
sowie unter Verbindungen der Formel:

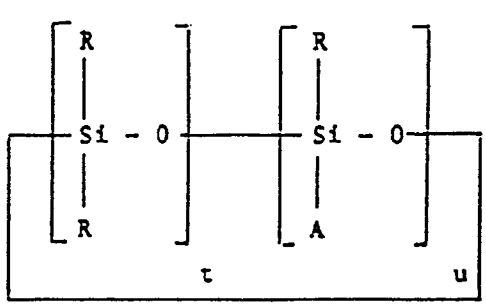

(2)

worin
R die gleichen Bedeutungen besitzt wie oben für die Formel (1) angegeben,
u eine Zahl zwischen 1 und 20 einschließlich bedeutet,
t eine Zahl zwischen 0 und 20 einschließlich bedeutet,

t + u gleich oder größer als 3 ist, wobei in den obigen Formeln A für einen Rest der Formel:

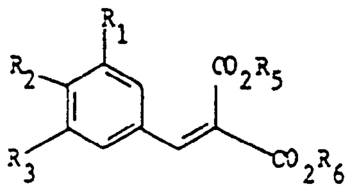

(3)

steht, worin $R_1$ und $R_2$ ausgewählt sind unter einem Wasstoffatom, einer Hydroxy-, Trimethylsilyloxy-, $C_1$-$C_6$-Alkyl-, $C_1$-$C_6$-Alkoxygruppe und einer divalenten Gruppe Y der Formel:

$$-(O)_n-(CH_2)_p-\underset{R_4}{\underset{|}{CH}}-CH_2-$$

worin n für 0 oder 1 steht,
p eine ganze Zahl zwischen 1 und 10 einschließlich, vorzugsweise zwischen 1 und 4, bedeutet und $R_4$ ausgewählt ist unter einem Wasserstoffatom und einer $C_1$-$C_4$-Alkylgruppe, wobei einer der Reste $R_1$ und $R_2$ für die Gruppe Y stehen muß,
$R_3$ ein Wasserstoffatom, eine $C_1$-$C_6$-Alkyl- oder $C_1$-$C_6$-Alkoxygruppe bedeutet,
$R_5$ und $R_6$, die gleich oder verscheiden sein können, eine $C_1$-$C_8$-Alkylgruppe bedeutet,
in der Kosmetik als Filter für UV-Strahlen mit einer Wellenlänge im Bereich von 280 und 360 nm.

4. Verwendung von statistischen Polymeren oder Blockpolymeren der Diorganopolysiloxane der Formeln (1) oder (2) nach Anspruch 3, die wenigstens eines der folgenden Charakteristika aufweisen:
R bedeutet Methyl,
B bedeutet Methyl,
$R_1$ bedeutet H oder Y,
$R_2$ bedeutet Y, Methoxy oder Butoxy,
$R_3$ bedeutet H oder Methoxy,
n = 0 oder 1,
p = 1,
$R_4$ bedeutet H oder Methyl,
$R_5$ und $R_6$ bedeuten Ethyl oder 2-Ethylhexyl,
r liegt zwischen 5 und 20 einschließlich,
s liegt zwischen 2 und 15 einschließlich,
t + u liegt zwischen 3 und 10 einschließlich,
in der Kosmetik als Filter für für UV-Strahlen mit einer Wellenlänge im Bereich von 280 und 360 nm.

5. Verwendung eines Polydimethylsiloxans mit einer Benzalmalonatfunktion der Formel (1) nach Anspruch 1, worin R und B für Methyl stehen, r = 5, s = 5, $R_1$ für H steht, $R_2$ für Y steht, wobei n = 1, p = 1 und $R_4$ für H steht, $R_3$ für H oder Methoxy steht und $R_5$ und $R_6$ für Ethyl stehen, in der Kosmetik als Filter für UV-Strahlen mit einer Wellenlänge im Bereich von 280 und 360 nm.

6. Verwendung eines Polydimethylsiloxans mit einer Benzalmalonatfunktion der Formel (1) nach Anspruch 1, worin R und B für Methyl stehen, r = 20, s = 5, $R_1$ für H steht, $R_2$ für Y steht, wobei n = 1, p = 1 und $R_4$ für H steht, $R_3$ für H steht und $R_5$ und $R_5$ für Ethyl stehen, in der Kosmetik als Filter für UV-Strahlen mit einer Wellenlänge im Bereich von 280 und 360 nm.

7. Verwendung eines Polydimethylsiloxans mit einer Benzalmalonatfunktion der Formel (1) nach Anspruch 1, worin R und B für Methyl stehen, r = 0, s = 40, $R_1$ für H steht, $R_2$ für Y steht, wobei n = 1, p = 1 und $R_4$ für H steht, $R_3$ für H steht und $R_5$ und $R_5$ für Ethyl stehen, in der Kosmetik als Filter für UV-Strahlen mit einer Wellenlänge im Bereich von 280 und 360 nm.

8. Verwendung eines Polydimethylsiloxans mit einer Benzalmalonatfunktion der Formel (1) nach Anspruch 1, worin R und B für Methyl stehen, r = 7,3, s = 9, $R_1$ für H steht, $R_2$ für Y steht, wobei n = 1, p = 1 und $R_4$ für $CH_3$ steht, $R_3$ für H steht und $R_5$ und $R_6$ für Ethyl stehen, in der Kosmetik als Filter für UV-Strahlen mit einer Wellenlänge im Bereich von 280 und 360 nm.

9. Verwendung eines Polydimethylsiloxans mit einer Benzalmalonatfunktion der Formel (1) nach Anspruch 1, worin R und B für Methyl stehen, r = 5, s = 5, $R_1$ für Y steht, wobei n = 0, p = 1 und $R_4$ für H steht, $R_2$ für Methoxy steht, $R_3$ für H oder Methoxy steht und $R_5$ und $R_6$ für Ethyl stehen, in der Kosmetik als Filter für UV-Strahlen mit einer Wellenlänge im Bereich von 280 und 360 nm.

10. Verwendung eines Polydimethylsiloxans mit einer Benzalmalonatfunktion der Formel (1) nach Anspruch 1, worin R und B für Methyl stehen, r = 5, s = 5, $R_1$ für Y steht, wobei n = 0, p = 1 und $R_4$ für H steht, $R_2$ für Methoxy steht, $R_3$ für Methoxy steht und $R_5$ und $R_5$ für 2-Ethylhexyl stehen, in der Kosmetik als Filter für UV-Strahlen mit einer Wellenlänge im Bereich von 280 und 360 nm.

11. Verwendung eines Polydimethylsiloxans mit einer Benzalmalonatfunktion der Formel (1) nach Anspruch 1, worin R und B für Methyl stehen, r = 5, s = 5, $R_1$ für Y steht, wobei n = 0, p = 1 und $R_4$ für H steht, $R_2$ für Butoxy steht, $R_3$ für Methoxy steht und $R_5$ und $R_6$ für Ethyl stehen, in der Kosmetik als Filter für UV-Strahlen mit einer Wellenlänge im Bereich von 280 und 360 nm.

12. Kosmetisches Mittel, dadurch gekennzeichnet, daß es in einem kosmetisch verträglichen Träger eine wirksame Menge wenigstens eines Diorganopolysiloxans mit einer Benzalmalonatfunktion enthält, das ausgewählt ist unter Verbindungen der Formel:

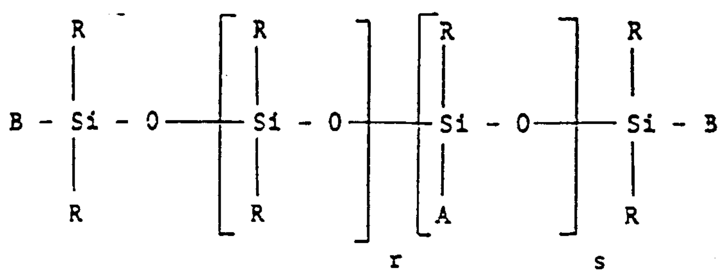

(1)

worin
die Reste R, die gleich oder verschieden sein können, ausgewählt sind unter $C_1$-$C_{10}$-Alkyl, Phenyl und 3,3,3-Trifluorpropyl, wobei wenigstens 80% der Reste R einen Methylrest bedeuten,
die Reste B, die gleich oder verschieden sein können, ausgewählt sind unter den Resten R und dem Rest A,
r eine Zahl zwischen 0 und 200 einschließlich ist,
s eine Zahl zwischen 0 und 50 einschließlich ist, und
wenn s für 0 steht, wenigstens einer der Reste B für A steht,
sowie unter Verbindungen der Formel:

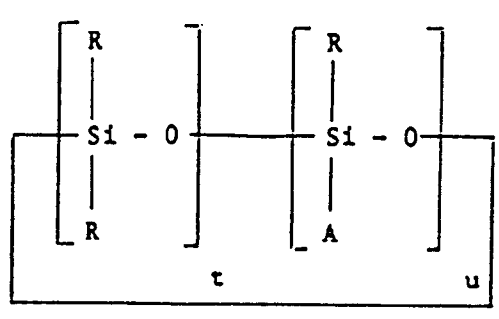

(2)

worin
R die gleichen Bedeutungen besitzt wie oben für die Formel (1) angegeben,

u eine Zahl zwischen 1 und 20 einschließlich bedeutet,
t eine Zahl zwischen 0 und 20 einschließlich bedeutet,
t + u gleich oder größer als 3 ist,
wobei in den obigen Formeln A für einen Rest der Formel:

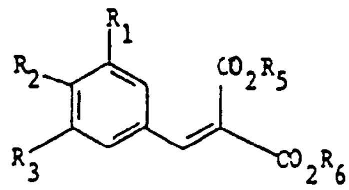

(3)

steht, worin $R_1$ und $R_2$ ausgewählt sind unter einem Wasstoffatom, einer Hydroxy-, Trimethylsilyloxy-, $C_1$-$C_6$-Alkyl-, $C_1$-$C_6$-Alkoxygruppe und einer divalenten Gruppe Y der Formel:

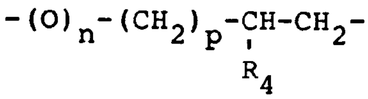

worin n für 0 oder 1 steht,
p eine ganze Zahl zwischen 1 und 10 einschließlich bedeutet und $R_4$ ausgewählt ist unter einem Wasserstoffatom und einer $C_1$-$C_4$-Alkylgruppe, wobei einer der Reste $R_1$ und $R_2$ für die Gruppy Y stehen muß,
$R_3$ ein Wasserstoffatom, eine $C_1$-$C_6$-Alkyl- oder $C_1$-$C_6$-Alkoxygruppe bedeutet,
$R_5$ und $R_6$, die gleich oder verschieden sein können, eine $C_1$-$C_8$-Alkylgruppe bedeutet.

**13.** Kosmetisches Mittel nach Anspruch 12, dadurch gekennzeichnet, daß es ein statistisches oder Blockpolymer eines Diorganopolysiloxans mit einer Benzalmalonatfunktion der Formel (1) oder (2) enthält, das wenigstens eines der folgenden Charakteristika aufweist:
R bedeutet Methyl,
B bedeutet Methyl,
$R_1$ bedeutet H oder Y,
$R_2$ bedeutet Y, Methoxy oder Butoxy,
$R_3$ bedeutet H oder Methoxy,
n = 0 oder 1,
p = 1,
$R_4$ bedeutet H oder Methyl,
$R_5$ und $R_6$ bedeuten Ethyl oder 2-Ethylhexyl,
r liegt zwischen 5 und 20 einschließlich,
s liegt zwischen 2 und 15 einschließlich,
t + u liegt zwischen 3 und 10 einschließlich.

**14.** Kosmetisches Mittel nach Anspruch 12 oder 13, dadurch gekennzeichnet, daß es ein Polydimethylsiloxan mit einer Benzalmalonatfunktion der Formel (1) nach Anspruch 1 enthält, worin R und B für Methyl stehen, r = 5, s = 5, $R_1$ für H steht, $R_2$ für Y steht, wobei n = 1, p = 1 und $R_4$ für H steht, $R_3$ für H oder Methoxy steht und $R_5$ und $R_5$ für Ethyl stehen.

**15.** Kosmetisches Mittel nach Anspruch 12 oder 13, dadurch gekennzeichnet, daß es ein Polydimethylsiloxan mit einer Benzalmalonatfunktion der Formel (1) nach Anspruch 1 enthält, worin R und B für Methyl stehen, r = 20, s = 5, $R_1$ für H steht, $R_2$ für Y steht, wobei n = 1, p = 1 und $R_4$ für H steht, $R_3$ für H steht und $R_5$ und $R_5$ für Ethyl stehen.

**16.** Kosmetisches Mittel nach Anspruch 12 oder 13, dadurch gekennzeichnet, daß es ein Polydimethylsiloxan mit einer Benzalmalonatfunktion der Formel(1) nach Anspruch 1 enthält, worin R und B für Methyl stehen, r = 0, s = 40, $R_1$ für H steht, $R_2$ für Y steht, wobei n = 1, p = 1 und $R_4$ für H steht, $R_3$ für H steht und $R_5$ und $R_5$ für Ethyl stehen.

**17.** Kosmetisches Mittel nach Anspruch 12 oder 13, dadurch gekennzeichnet, daß es ein Polydimethylsiloxan mit einer Benzalmalonatfunktion der Formel (1) nach Anspruch 1 enthält, worin R und B für Methyl stehen, r = 7,3, s = 9, $R_1$ für H steht, $R_2$ für Y steht, wobei n = 1, p = 1 und $R_4$ für $CH_3$ steht, $R_3$ für H steht und $R_5$ und $R_5$ für Ethyl stehen.

**18.** Kosmetisches Mittel nach Anspruch 12 oder 13, dadurch gekennzeichnet, daß es ein Polydimethylsiloxan mit einer Benzalmalonatfunktion der Formel (1) nach Anspruch 1 enthält, worin R und B für Methyl stehen, r = 5, s = 5, $R_1$ für Y steht, wobei n = 0, p = 1 und $R_4$ für H steht, $R_2$ für Methoxy steht, $R_3$ für H oder Methoxy steht und $R_5$ und $R_5$ für Ethyl stehen.

**19.** Kosmetisches Mittel nach Anspruch 12 oder 13, dadurch gekennzeichnet, daß es ein Polydimethylsiloxan mit einer Benzalmalonatfunktion der Formel(1) nach Anspruch 1 enthält, worin R und B für Methyl stehen, r = 5, s = 5, $R_1$ für Y steht, wobei n = 0, p = 1 und $R_4$ für H steht, $R_2$ für Methoxy steht, $R_3$ für Methoxy steht und $R_5$ und $R_5$ für 2-Ethylhexyl stehen.

**20.** Kosmetisches Mittel nach Anspruch 12 oder 13, dadurch gekennzeichnet, daß es ein Polydimethylsiloxan mit einer Benzalmalonatfunktion der Formel (1) nach Anspruch 1 enthält, worin R und B für Methyl stehen, r = 5, s = 5, $R_1$ für Y steht, wobei n = 0, p = 1 und $R_4$ für H steht, $R_2$ für Butoxy steht, $R_3$ für Methoxy steht und $R_5$ und $R_5$ für Ethyl stehen.

**21.** Kosmetisches Mittel nach einem der Ansprüche 12 bis 20, dadurch gekennzeichnet, daß es zusätzlich kosmetische Adjuvantien enthält, die ausgewählt sind unter Verdickungsmitteln, weichmachenden Mitteln, Befeuchtungsmitteln, Tensiden, Konservierungsmitteln, Antischaummitteln, Parfüms, Ölen, Wachsen, Lanolin, niedrigen Monoalkoholen und Polyalkoholen, Benzoaten von $C_{12}$-$C_{15}$-Alkoholen, Treibmittlen, Farbstoffen und Pigmenten.

**22.** Mittel nach einem der Ansprüche 12 bis 21, dadurch gekennzeichnet, daß es als ölige, alkoholische oder ölig-al-koholische Lotion, Emulsion, ölig-alkoholisches. alkoholisches oder wäßrig-alkoholisches Gel, fester Stift, Spray oder Aerosol vorliegt.

**23.** Kosmetisches Mittel nach einem der Ansprüche 12 bis 22, dadurch gekennzeichnet, daß es ein Mittel zum Schutz der menschlichen Epidermis darstellt und 0,25 - 3 Gew.-% eines Diorganopolysiloxans der Formel (1) oder (2) enthält.

**24.** Kosmetisches Mittel nach einem der Ansprüche 12 bis 22 in Form eines Sonnenschutzmittels, dadurch gekennzeichnet, daß es 0,5 - 15 Gew.-% eines Diorganopolysiloxans der Formel (1) oder (2) enthält.

**25.** Kosmetisches Sonnenschutzmittel nach Anspruch 24, dadurch gekennzeichnet, daß es zusätzlich einen Filter für UV-B- und/oder UV-A-Strahlen enthält.

**26.** Kosmetisches Mittel nach einem der Ansprüche 12 bis 21 zum Auftragen auf das Haar, dadurch gekennzeichnet, daß es in Form eines Shampoos, einer Lotion, eines Gels oder einer Emulsion zum Spülen, in einer Form zum Auftragen vor oder nach dem Shampoonieren, vor oder nach dem Färben oder Entfärben, vor oder nach Legen einer Dauerwelle, als Frisier- oder Behandlungslotion oder -gel, als Lotion oder Gel zum Bürsten oder zum Legen einer Wasserwelle , Frisierspray oder Haarlack vorliegt und 0,25 - 5 Gew.-% eines Diorganopolysiloxans der Formel (1) oder (2) enthält.

**27.** Kosmetisches Mittel nach einem der Ansprüche 12 bis 21 in Form eines gefärbten oder nicht-gefärbten kosmetischen Mittels, dadurch gekennzeichnet, daß es ein Haarmittel, ein Schminkprodukt oder ein Mittel zur Pflege oder Behandlung der Haut darstellt, das 0,25 - 3 Gew.-% eines Diorganopolysiloxans der Formel (1) oder (2) enthält.

**28.** Kosmetisches Verfahren zum Schutz der Haut und natürlicher oder sensibiliserter Haare vor UV-Strahlung, dadurch gekennzeichnet, daß man auf die Haut oder die Haare eine wirksame Menge eines kosmetischen Mittels aufträgt, das wenigstens ein Diorganopolysiloxan mit einer Benzalmalonatfunktion der Formel (1) oder (2) wie in einem der Ansprüche 3 bis 11 definiert, enthält.

**29.** Verfahren zum Schutz eines kosmetischen Mittels vor UV-Strahlen, dadurch gekennzeichnet, daß man dem Mittel eine wirksame Menge wenigstens eines Diorganopolysiloxans mit einer Benzalmalonatfunktion der Formel (1) oder (2), wie in einem der Ansprüche 3 bis 11 definiert, einverleibt.